(19)

**Europäisches Patentamt**
**European Patent Office**
**Office européen des brevets**

(11) **EP 3 731 238 A1**

(12) **EUROPEAN PATENT APPLICATION**

(43) Date of publication:
**28.10.2020 Bulletin 2020/44**

(21) Application number: **19170581.3**

(22) Date of filing: **23.04.2019**

(51) Int Cl.:
**G16H 50/30** (2018.01)    **A61B 5/11** (2006.01)
**G16H 50/70** (2018.01)    **G16H 20/30** (2018.01)
**G16H 20/10** (2018.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **CENTRE NATIONAL DE LA RECHERCHE SCIENTIFIQUE**
**75016 Paris (FR)**
• **Université Paris Descartes - Paris V**
**75006 Paris (FR)**
• **Univ Paris XIII Paris-Nord Villetaneuse**
**93430 Villetaneuse (FR)**
• **Etat Français - Ministère de la Défense - Direction centrale du service de santé des armées**
**75614 Paris Cedex 12 (FR)**
• **École Normale Supérieure Paris-Saclay**
**94230 Cachan (FR)**

(72) Inventors:
• **VIDAL, Pierre Paul**
**75005 PARIS (FR)**
• **VIENNE-JUMEAU, Aliénor**
**78150 LE CHESNAY (FR)**
• **RICARD, Damien**
**92140 CLAMART (FR)**
• **OUDRE, Laurent**
**75012 PARIS (FR)**
• **VAYATIS, Nicolas**
**75005 PARIS (FR)**
• **MOREAU, Albane**
**92140 CLAMART (FR)**
• **QUIJOUX, Flavien**
**75020 PARIS (FR)**

(74) Representative: **Dennemeyer & Associates S.A.**
**Postfach 70 04 25**
**81304 München (DE)**

(54) **DEVICE FOR CALCULATING, DURING ONE STEP OR EACH SUCCESSIVE STEP OF THE GAIT OF A SUBJECT, THE PUSH-OFF OF THE SUBJECT**

(57)    The present invention presents a device for calculating, during one step or each successive step of the gait of a subject, the push-off $P_0$ of the subject, which is the power per kilogram released by the ankle push-off moment, which comprises: at least one inertial measurement unit (1A, 1B) on one foot of the subject, the inertial measurement unit (1A, 1B) having: at least one accelerometer to measure the vertical and antero-posterior accelerations and/or at least one gyroscope to measure the medio-lateral angular speed data $\dot{\alpha}$ during the gait, calculation means (2A) connected to the Inertial measurement unit (1A, 1B), configured to calculate: for the foot, and for the step or each successive step of the gait: - the time of the heel-off and the time of the toe-off, - the push-off $P_0$, by the Euler's equation stating that the sum of moments acting on the foot taken as a rigid body, being equal to the rate of change of the angular momentum of the foot, with the calculation of the push-off $P_0$ at the time of the toe-off where the sagittal angular momentum is at its maximum in absolute value, displaying means (2B) connected to the calculation means (2A).

FIG. 1A

**Description**

FIELD OF THE INVENTION

The invention concerns a gait quantification device for calculating the push-off $P_0$ of a subject, the push-off $P_0$ being the power per kilogram released by the ankle push-off moment.

STATE OF ART

Walking is one of the most automatic activities people do, until it gets altered. Then, it becomes highly intricate to recover due to its high complexity. Gait impairments are a major risk factor of falls, which represent a high burden in this population. Indeed, falls can be medically injurious and result in acute lesions such as fractures, traumatic brain injury or even renal insufficiency, especially in elderlies or patients with comorbidities such as Multiple Sclerosis patients. They also bear chronic consequences such as fear of falling and repeated falls, which lead the individuals to curtail their activity, resulting in physiologic deconditioning, loss of independence, and institutionalization.

Early signs of abnormal gait can sometimes occur with gradual intensity for several years before any clinical diagnosis, as has recently been described about imbalance among Parkinson patients for instance, and smoothness among multiple sclerosis (MS) patients. Indeed, the effect of exercise on gait has been documented in several diseases. Nonetheless, the more specific and early the rehabilitation measures, the slower the gait deterioration. Therefore, the early signs of should be intensively looked for, to ensure immediate and appropriate intervention.

To detect such signs, it is essential that normal gait be minutely and quantitatively described. Such attentions make it possible to identify meaningful perturbations resulting from the disease, as opposed to normal variants. Today, this evaluation relies mostly on the naked instructed eye of the neurologist. This evaluation remains subjective and the ordinal scores of these semi-quantitative clinical data are bound to be insufficient after diagnosis, when the improvement or deterioration need to be evaluated during the medical follow-up. The objective description and quantitative assessment of gait appear complementary to the clinical and systematic evaluation of gait dysfunctions.

In that context, further instrumental objective investigations of gait in patients with gait impairments is of interest to screen for falls and follow-up on the disease evolution. Gait speed data is a valuable index. However, it is not causally related to falls, and promoting an increase in walking velocity may disrupt an adaptive process that allows the patient to be precautious.

Another feature available to characterize gait is the peak of the shortening contractions of the plantar flexors, the soleus and the gastrocnemius muscles during the late stance, known as "push-off" (Po). It is also related to the elastic recoil of the Achilles tendon. Push-off is decreased in various pathologies, either neurological (such as multiple sclerosis, Parkinson, peripheral neuropathies, normal pressure hydrocephaly...) or not (frailty, arthrosis, limb amputation...). In multiple sclerosis for instance, the spasticity decreases push-off because of a lower contraction activation of the soleus and gastrocnemius muscles. The situation decreases the toe clearance during the initial swing and the dragging leads to tripping. Moreover, push-off of the support limb during tripping can help recovery by providing time and clearance for adequate positioning of the swinging foot and by restraining the angular momentum of the body during push-off.

Various techniques are implemented to measure push-off, which may include video analysis, optokinetic recording, registration of muscle contractions by electromyogram or the reaction of the ground in contact with the person through force platforms.

A combination of these measurement tools is also possible. Kempen et al. (2016) [1] used force platforms to measure the magnitude of the push-off force in Newton to show a combined decrease in heel-rise and step-clearance during the swing phase in patients with multiple sclerosis.
The decrease in push-off may be associated with a decrease in knee extension at the end of the swing phase and an increase in hip flexion at the beginning of the swing phase. These results are found by Filli et al. 2018 [2] who make the suggestion to group multiple sclerosis patients according to their knee and ankle control disorders.

A few years ago, Kelleher et al. (2010) [3] also showed a decrease in the propulsive force and a reduction in the ankle plantar-flexion angle in these same patients. This push-off measurement was performed by a combination of electromyogram, force platforms and an optokinetic system, which is the usual approach for laboratory analysis. However, these current methods of estimating the push-off are not suitable for ambulatory measurement.

Using force plates (the gold-standard) is not satisfying because:

- it requires the patient to perform several U-turns during walking to make sure enough steps are recorded on the force-plates. In particular, this is difficult for patients with fatigability such as multiple sclerosis patients;
- force plates are expensive;
- force plates are heavy and cumbersome: as such, they are not easily brought to the patient in clinical settings. This is why measures on force plates need the patient to come to the lab. As a consequence, measure in daily living are not possible using this technology;

- force plates require the patient to be careful while walking to make sure his foot falls on the force plates.

Electromyogram has its own withdrawals:

- It needs to clean the surface of application on the skin with can be both time consuming and uncomfortable;
- it requires a specific signal analysis with usually requires computing the maximal voluntary isometric contraction which is particularly difficult to measure in an ambulatory context and with neurological patients;
- the signal can be contaminated by electrical interferences, activity of other muscles and mechanical artefacts due to motion;
- it cannot record efficiently deep muscles such as Soleus and Tibialis posterior muscles, both involved in the push-off;
- body fat reduces the muscular signal recording which is an issue in patients with obesity.

Optokinetic and three-dimensional (3D) video cameras have their flaws too:

- Both are expensive (over tens of thousands of dollars) especially because these two technics needs several cameras and/or particularly complex spatial reconstruction;
- they require reconstructing the skeleton in order to compute the body parts and to compute their 3D coordinates;
- they need the use of several markers to put on the patient's body which is time consuming and a high source of variability between practitioners;
- a large volume of empty space without obstacle between the subject and the cameras is needed in order to record the body motion. This point makes them incompatible with the private or public medical practice.

SUMMARY OF THE INVENTION

Here, the invention presents a device for calculating a proxy of push-off during gait using inertial measurement units.

BRIEF DESCRIPTION OF THE DRAWINGS

Other advantages and characteristics of the disclosed device and method of the present invention will become apparent from reading the description, illustrated by the following figures, where:

Figure 1A: represents the IMU on the subject, the IMU being connected to a tablet.

Figure 1B: Free-body diagram of foot during late stance showing a simplified model for definition of the ankle push-off moment power (Po). $\vec{P}$ is the weight force and $\vec{F}$ is the force applied by extensor muscles at the Achille tendon.

Figure 2: Definition of Po.

A) The medio-lateral angular speed of the left and right feet is shown in dark grey and light grey, respectively. The arrows indicate left and right push-off points, with the y-axes giving the Po parameter. Corresponding phases of the gait cycles are represented schematically below.

B) Computation of Po.

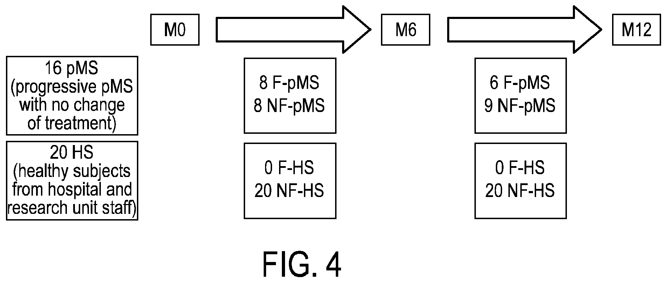

Figure 4: Flowchart of the inclusion and follow-up of both cohorts in the clinical study. M, month; pMS-F, pMS fallers; pMS-NF, pMS non-fallers; HS, healthy subjects.

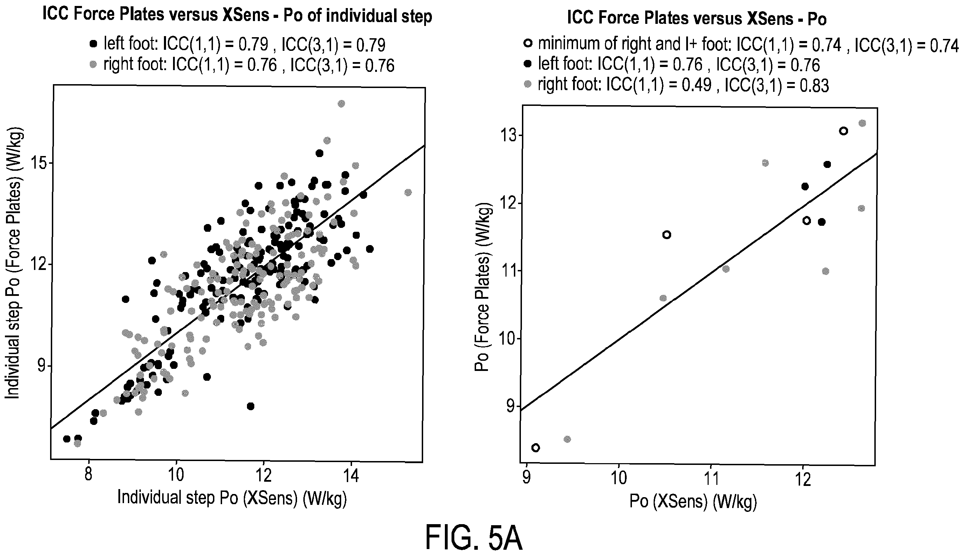

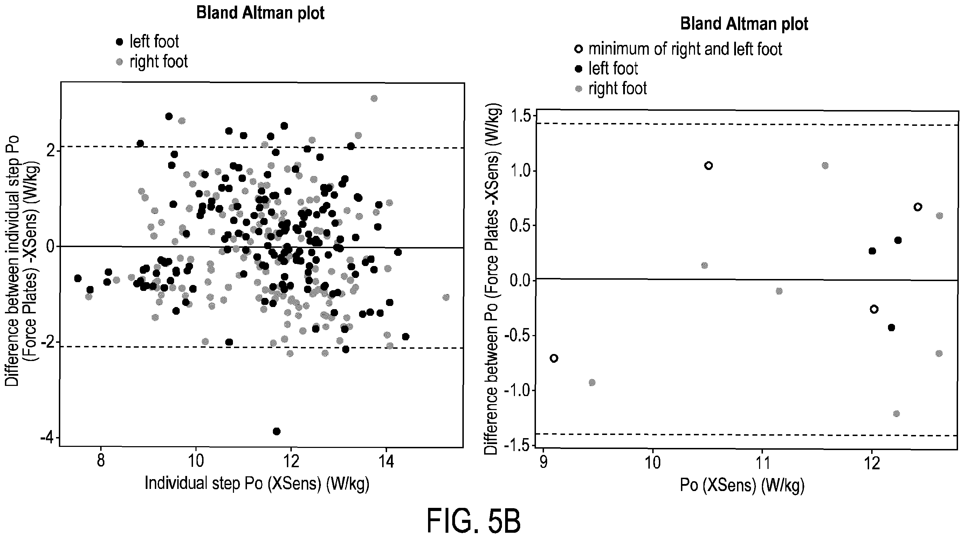

Figure 5: Tests of Po validity (comparison of Po as measured with Xsens® and force plates)

A) Scatterplot of the Po as measured with force plates versus Xsens® for individual steps (left panel) and the whole walk (right panel).
B) Bland Altman plot for Po as measured with force plates versus Xsens® for individual steps (left panel) and the whole walk (right panel).

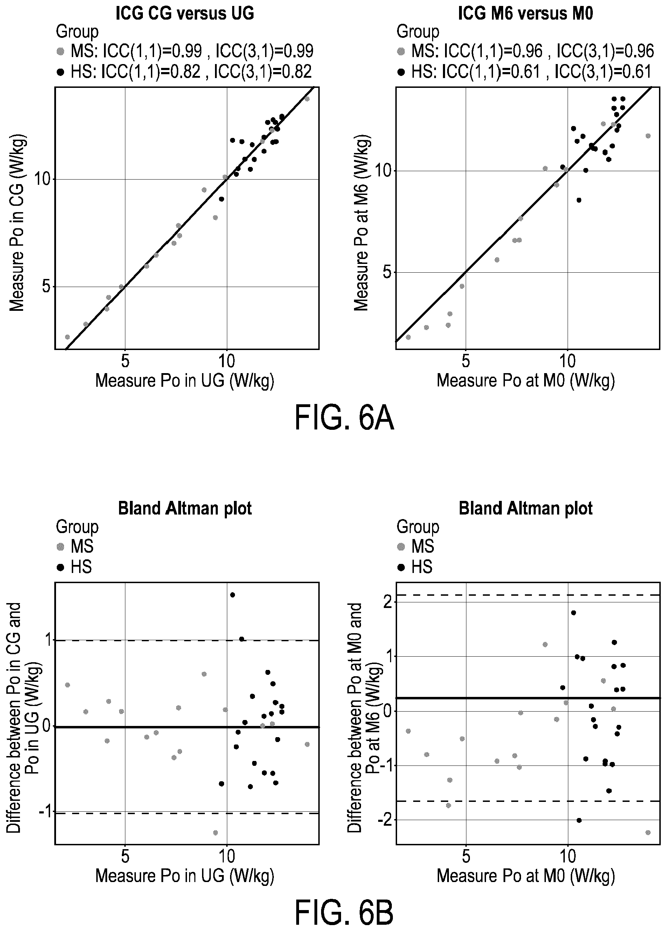

Figure 6: Tests of Po reproducibility (comparison of Po under unconstrained gaze [UG] and constrained gaze [CG] conditions) and repeatability (comparison of Po at different times: M0 and M6).

A) Scatterplot of the Po under CG versus UG (left panel, reproducibility test) and at M6 versus M0 (right panel, repeatability test)

B) Bland Altman plot for the reproducibility test (left panel) and repeatability at 6 months (right panel)

Figure 7:

A) Comparison of Po in the 3 cohorts: progressive multiple sclerosis (pMS) with and without falls (pMS-F), pMS-NF and healthy subjects (HS).

B) Receiver operating characteristic (ROC) curves for the training cohort involving 10 people with pMS, ie 10 measurements at M0 and 10 measurements at M6, (left box) and the test cohort 6 people with pMS, ie 6 measurements at M0 and 6 measurements at M6, (right box). Cutoffs were determined with the training cohort and their predictive values were computed with the test cohort. Dashed curves are ROC curves for each configurations of the Monte Carlo cross-validation (and the nested 4-fold cross-validation for the training set). Plain curves are means of all dashed curves. The curves are for Po and the timed 25-foot walk test.

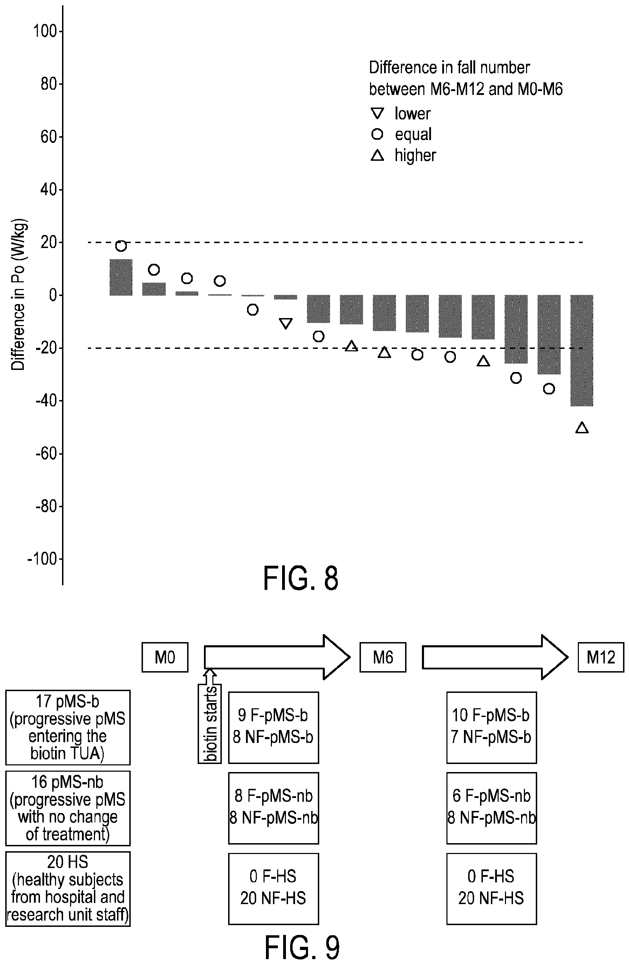

Figure 8: Waterfall plot showing the difference in Po between M0 and M6 in pMS patients. The dashed red lines limit the zone of significant change (-20% and +20%) for Po. The cohort showed no increase of > 20% in Po.

Figure 9: Flowchart of the inclusion and follow-up of the three cohorts.

Figure 10:

A) Waterfall plot showing the difference in Po between M0 and M6 in pMS-b patients. The dashed horizontal lines limit the zone of significant change (-20% and +20%) for Po. R: complete responders.
B) Waterfall plot showing the difference in Po between M0 and M6 in pMS-nb patients. The dashed horizontal lines limit the zone of significant change (-20% and +20%) for Po. No complete responders are found in the cohort.

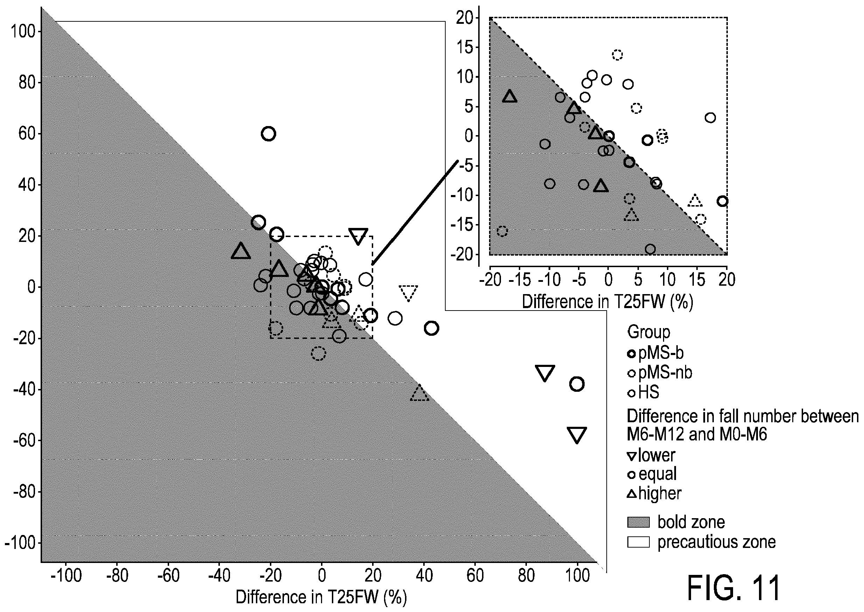

Figure 11: Difference in Po according to the difference in T25FW (Timed 25 Foot Walk) between M0 and M6 in the three pMS-b, pMS-nb and HS cohorts. The dashed horizontal lines limit the zone of significant change ($\geq$20%) for T25FW, and the equivalent change for Po. The diagonal dashed line delimits the bold zone (where the improvement in T25FW is greater than the improvement of Po or where the decrease in T25FW is lower than the decrease in Po) from the precautious zone (where the improvement in T25FW is lower than the improvement of Po or where the decrease in T25FW is greater than the decrease in Po).

## GENERAL DESCRIPTION

The present invention presents a device for calculating, during one step or each successive step of the gait of a subject, the push-off Po of the subject, which is the power per kilogram released by the ankle push-off moment.
The device comprises:

- at least one inertial measurement unit 1A, 1B on one foot of the subject, the inertial measurement unit 1A, 1B having: at least one accelerometer to measure the vertical and antero-posterior accelerations and/or at least one gyroscope to measure the medio-lateral angular speed data $\dot{\alpha}$ during the gait,
- calculation means 2A connected to the inertial measurement unit 1A, 1B, configured to calculate: for the foot, and for the step or each successive step of the gait:

  - the time of the heel-off and the time of the toe-off,

  - the push-off $P_0$, by the Euler's equation stating that the sum of moments acting on the foot taken as a rigid body, being equal to the rate of change of the angular momentum of the foot, with the calculation of the push-off $P_0$ at the time of the toe-off when the sagittal angular momentum is at its maximum in absolute value.

The device comprises also displaying means 2B connected to the calculation means 2A.

In one embodiment, the device comprises at least two inertial measurement units 1A, 1B, one inertial measurement unit 1A, 1B, per foot, each Inertial measurement unit 1A, 1B has at least one gyroscope to measure the medio-lateral angular speed data $\dot{\alpha}$ during the gait,
and the push-off $P_0$ is equal to :

$$\mathrm{Po} = r_A \, . \, g . \cos \alpha \, . \, \omega$$

$\alpha$ being the integration of the medio-lateral angular speed data $\dot{\alpha}$ between the time of the heel-off and the time of the toe-off,
$\omega$ being a value of the gyration medio-lateral angular speed data $\dot{\alpha}$,
$r_A$ being the distance between the center of pressure to the tibio-talus articulation.
$r_A$ can be extracted from existing reference tables validated in the literature of inverse dynamics.

Advantageously, $\omega$ is the value of the gyration medio-lateral angular speed data $\dot{\alpha}$, at the time of the toe-off, at $\pm 10\%$.
Preferably, the device comprises at least one inertial measurement unit 1A, 1B per foot of the subject, the calculation means 2A calculate the push off P0 for successive steps. The calculation means 2A calculate for each foot, with N natural number greater than or equal to two:

- $\alpha_i$, i varying from 1 to N successive steps of the gait;
- $\omega_i$, i varying from 1 to N successive steps of the gait;
- the mean $|\mathrm{Po}| = r_A . g . \cos|\alpha| . |\omega|$
  with $|\alpha|$ the mean of $\alpha i$, and $|w|$ the mean of $wi$;
  and the calculation means 2A calculate $P_0$ which is function of: the push-off of the right foot $|P_{0\,right}|$ and the push-off of the left foot $|P_{0\,left}|$.

In one realization, $P_0$ is the minimum of $|P_{0\,right}|$ and $|P_{0\,left}|$.
N can be comprised between to 5 to 20, for a 10-meter walking exercise.
In one first embodiment, the device is intended to be embedded on the subject, the calculation means 2A and the displaying means 2B being an embedded support 3 which communicates with the inertial measurement unit 1A, 1B using for instance wireless communication such as WIFI or Bluetooth®.
The embedded support 3 can be a computer, a tablet, a smartphone with an Application for gait quantification.
In one second embodiment, the calculation means 2A and the displaying means 2B are physically separated, and are embedded or not on the subject, and communicate with the inertial measurement unit 1A, 1B with wire or wireless communication.
The present invention concerns also a system which comprises:

- the device for calculating the push-off $P_0$ previously described,
- a rehabilitation device of the subject linked to the device for calculating the push-off $P_0$ previously described.

The rehabilitation device of the subject can be:

- Functional Electrical Stimulation technologies ;
- Mechanical and electronical ankle-foot orthosis ;
- Robotic prosthesis and exoskeleton ;
- Biofeedback electrical device ;
- Connected insoles, shoes, socks or elastic supportive hoses;
- Continuous Passive Motion medical devices

The present invention concerns also a method to calculate, during one step or each successive step of the gait of a patient, the push-off $P_0$ of the subject, from the vertical and antero-posterior accelerations data and/or the medio-lateral angular speed data $\dot{\alpha}$ of the patient for one step or several successive steps of a gait, and by using the device for calculating the push-off $P_0$ previously described.
This method presents the following steps:

- (i) calculating the time of the heel-off and the time of the toe-off of the step or each successive step;
- (ii) calculating for the foot, the push-off $P_0$,

by the Euler's equation stating that the sum of moments acting on the foot taken as a rigid body, being equal to the rate of change of the angular momentum of the foot, with the calculation of the push-off $P_0$ at the time of the toe-off when the sagittal angular momentum is at its maximum in absolute value.

In one embodiment, in the step (ii), the method calculates for each foot, with N natural number greater than or equal to two:

- $\alpha_i$, i varying from 1 to N successive steps of the gait;
- $\omega_i$, i varying from 1 to N successive steps of the gait;
- the mean $|Po| = r_A.g.\cos|\alpha|.|\omega|$
  with $|\alpha|$ the mean of $\alpha i$, and $|w|$ the mean of $wi$;
- and $P_0$ which is function of: the push-off of the right foot $|P_{0\ right}|$ and the push-off of the left foot $|P_{0\ left}|$.

The method can be used to analyse/predict fall risk in patient, and comprises an additional step:

- (iii) with the value of the push-off $P_0$ compared to a threshold, displaying the fall risk of the patient, the fall risk being high compared to the fall risk of a reference healthy subject if the value of the push-off $P_0$ is inferior to the threshold.

In the step (iii), the threshold can be fixed with several patients so as to:

- maximize (sensitivity + specificity -1) (which is the Youden index);
- with the constraint that the sensitivity is superior to 90%;

In one embodiment, in the step (iv), the threshold is 6.9 W/kg for patients with multiple sclerosis. With this threshold, the error on the prediction of fall risk (1-accuracy where accuracy is equal to prevalence * (sensitivity) + (1 - prevalence)* specificity, with a prevalence of 50%) is 11.7%.

In another embodiment, the threshold is a previous value of the push-off of the patient calculated 6 months before, and a change of ≥20% is considered significant for all populations. For multiple sclerosis patient, a change of ≥0.53 W/kg is also considered significant. For healthy subjects, a change of ≥0.07 W/kg is also considered significant.

The method can be used to evaluate a treatment, by calculating, during each successive step of the gait of patients who follow the treatment, and comprises the additional step of (iii) display the time evolution of the push-off the patients.

An increase in the push-off $P_0$ means a decreaseof the fall risk of the patient, the treatment device being validated if the increase of the push-off $P_0$ is superior or equal to 20% .

A decrease in the push-off $P_0$ means an increase of the fall risk of the patient, the treatment device being unvalidated if the decrease of the push-off $P_0$ is inferior or equal to 20%

The treatment can be realized with a medication and/or a rehabilitation device and/or a rehabilitation program

ONE EXAMPLE

**Methods:**

Patients

*Biomechanical study:* Seven young healthy subjects (HS) were recruited from the university staff and enrolled in the preliminary study. The inclusion criteria included no report of falls in the 5 years before inclusion and no disease that could affect walking.

*Clinical study:* 16 patients with pMS and 20 sex- and age- matched HS were enrolled in this longitudinal prospective study (Table 1). pMS patients were consecutively recruited from the outpatient clinic of Percy Hospital (Clamart, France) between December 2017 and April 2018. A total of 20 HS participants were recruited from the hospital and research unit staff between December 2017 and April 2018. The inclusion criteria for the pMS cohort was age at least 18 years, a diagnosis of primary progressive or secondary progressive MS according to the 2010 International Panel criteria [34], capable of walking 20 m with a U-turn, and free of any other conditions that affect gait. Exclusion criteria were pregnancy and drug intake modification for the 6 months before inclusion or during the 12-month follow-up. The inclusion criteria for the HS cohort were no report of falls in the past 5 years before inclusion and no disease that could affect walking. All participants gave their written consent to participate in the study. The study protocol was conducted according to the Helsinki principles and approved by Ethics Committee "Protection des Personnes Nord Ouest III" under the ID RCB: 2017-A01538-45.

Measures

*Biomechanical study:* HS wore two 3-D accelerometers (Mtw XSens®, 100-Hz sampling frequency) positioned on the dorsal part of both feet. The IMUs were synchronized with two force plates (Kistler®, 200-Hz sampling frequency) placed in the middle of a 10-m walkway. Participants walked in and out of this walkway for 12 go's and returns, until a total of 48 steps per individual on the platform had been recorded.

*Clinical study:* Gait was measured by using four 3-D accelerometers (Mtw XSens®, 100-Hz sampling frequency) positioned on the head, lower back (L4-L5 vertebrae) and dorsal part of both feet. Participants performed two walks of 20 m with a U-turn (10 m on the way in and 10 m on the way out). The test-retest reproducibility - amount of variation in repeated measures under different conditions - depending on the environment and cognitive load was tested by measuring gait under 2 conditions: unconstrained gaze (UG) and constrained gaze (CG). In the UG condition, the patient was not given any instructions regarding the gaze and the corridor walls were left empty. In the CG condition, the patient was asked to focus the gaze on a target placed at eye height at both ends of the corridor. Unless specified, comparability between groups were computed in the UG condition. Repeatability - amount of variation in repeated measures under the same conditions -was established by repeating the walking trials at month 0 (M0) and M6. A single-measure reliability model was applied because future clinical use is likely to require time-efficient protocols and thus only one measure per subject. As well, learning effect and increased fatigue during the second test would be problematic to take into account.

Disease severity was assessed by patient-reported outcomes (the Multiple Sclerosis Walking Scale-12 and the Fatigue Impact Scale) together with clinical evaluation (Expanded Disease Severity Scale [EDSS] and Computerized Speed Cognitive Test) and the 25FWT administered according to a validated standardized protocol.

At the first visit (M0), participants were asked for the number and circumstances of falls in the previous 6 months, with falls described as "an event which results in a person coming to rest unintentionally on the ground or other lower level, not as a result of a major intrinsic event (such as a stroke) or overwhelming hazard". They were asked to record any fall that might occur. Participants were seen at M3, M6 and M12 after the initial visit to evaluate the number of falls they had since this visit. Patients who fell within 6 months after a visit were considered fallers at that visit (pMS-F), and patients who did not fall were considered non-fallers (pMS-NF).

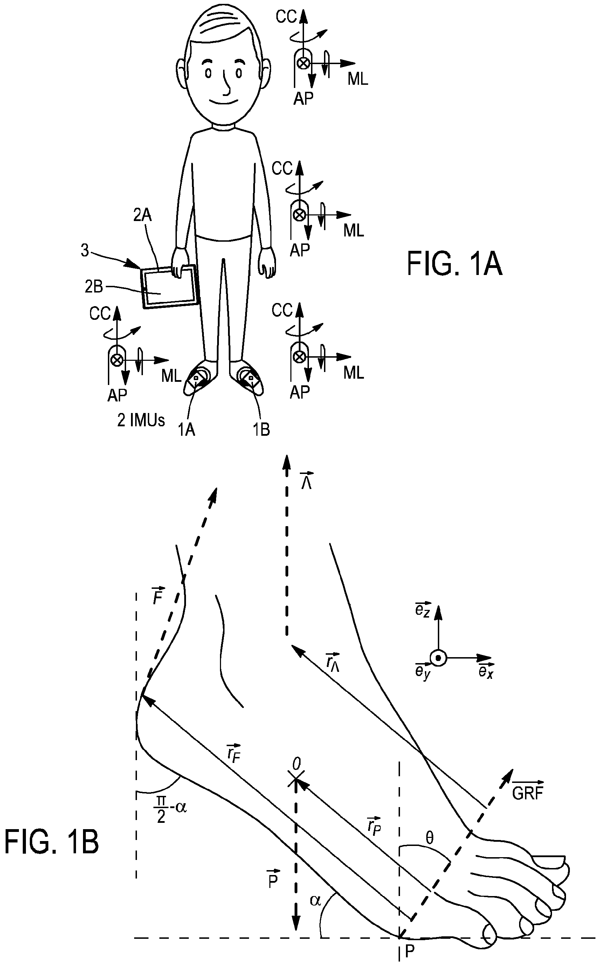

*Push-off estimation* (Figure 1B) - Using inverse dynamics on a highly simplified model extracted from the free body model with the foot taken as a solid, we defined a surrogate of the power per kilogram released by the ankle push-off moment (Po) as the product of the torque of muscle force per kilogram in the sagital plane ($C(\vec{F})$) and the joint angular velocity ($\omega = \dot{\alpha}$, with $\alpha$ being the angle between the floor and the plantar line) divided by body mass (m):

$$\mathrm{Po} = \frac{1}{m} . C(\vec{F}) \times \omega.$$

Euler's equation states that the sum of moments acting on the foot, taken as a rigid body, is equal to the rate of change of the angular momentum of the foot. At final contact (FC, also called toe-off because, in normal circumstances, the last segment of the foot to leave the ground are the toes), the sagittal angular momentum is at its minimum (in other words at its maximum in absolute value) (Catalfamo 2010, Formento 2014). Thus, the sum of moments acting on the foot is null:

$$(\vec{r_P} \wedge \vec{P}) . \vec{e_y} + (\vec{r_\Lambda} \wedge \vec{\Lambda}) . \vec{e_y} + C(\vec{F}) = 0$$

Where $\vec{r_P}$ is the vector joining the center of pressure to the center of mass at toe-off, $\vec{P}$ the weight of the foot, $\vec{r_\Lambda}$ the vector joining the center of pressure to the tibio-talus articulation center, $\vec{\Lambda}$ the bone-to-bone force ($\vec{\Lambda} = (m-m_f).g.\vec{e_z}$ with $m_f$ the mass of the foot and $g$ the standard gravity) and $\vec{e_y}$ a unit vector in the medio-lateral direction. We thus have:

$$C(\vec{F}) = -[(\overrightarrow{r_P} \wedge \vec{P}) + (\overrightarrow{r_A} \wedge \vec{A})].\overrightarrow{e_y}$$

ie
$$C(\vec{F}) = -[-m_f g\,(\overrightarrow{r_P} \wedge \overrightarrow{e_z}) + (m - m_f).g.\,(\overrightarrow{r_A} \wedge \overrightarrow{e_z})].\overrightarrow{e_y}$$

ie
$$C(\vec{F}) = m.g.\,[(\frac{m_f}{m}\overrightarrow{r_P} - \left(1 - \frac{m_f}{m}\right).\overrightarrow{r_A}) \wedge \overrightarrow{e_z}].\overrightarrow{e_y}$$

Where $\overrightarrow{r_P} \wedge \overrightarrow{e_z} = -r_P.\cos\alpha.\overrightarrow{e_Y}$ and $\overrightarrow{r_A} \wedge \overrightarrow{e_z} = -r_A.\cos\alpha.\overrightarrow{e_Y}$. We thus have:

$$C(\vec{F}) = m.g.\,[-\frac{m_f}{m}r_P.\cos\alpha + \left(1 - \frac{m_f}{m}\right).r_A.\cos\alpha]$$

ie
$$\frac{1}{m}.C(\vec{F}) = [(1 - \frac{m_f}{m}).r_A - \frac{m_f}{m}.r_P].g.\cos\alpha$$

ie
$$\frac{1}{m}.C(\vec{F})\,\cos\alpha \quad \text{as} \quad \frac{m_f}{m} \sim 0.01$$

and
$$\text{Po} = r_A.g.\cos\alpha.\omega \qquad\qquad (\text{eq. 1}),$$

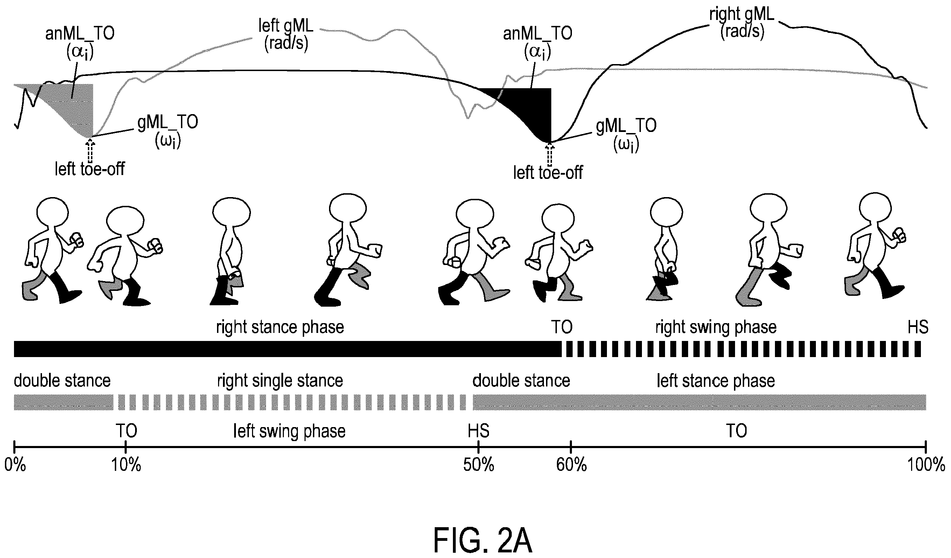

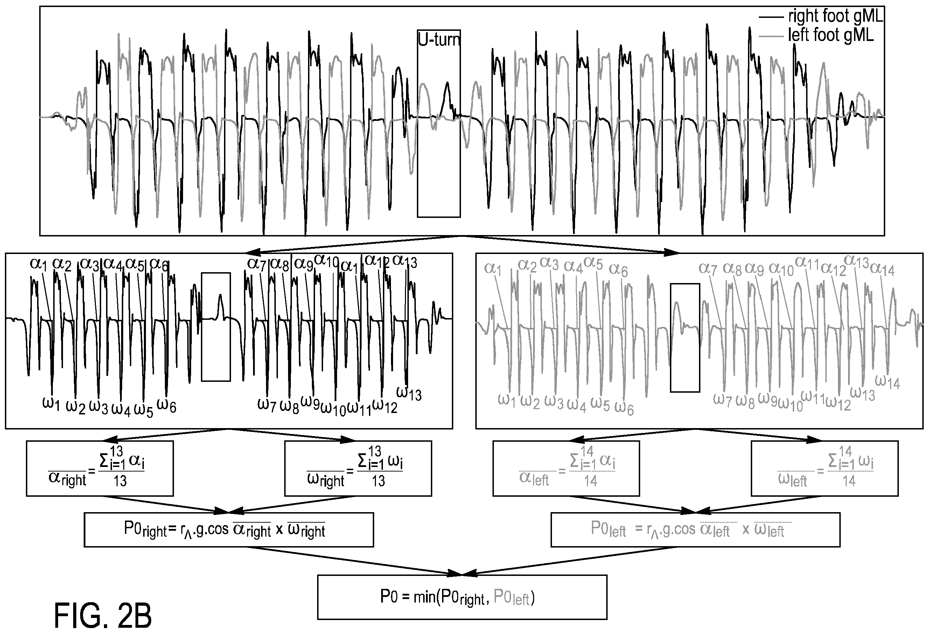

a surrogate of the power per kilogram released by the ankle push-off during late stance. IMUs give access to both $\dot\alpha$ and, by integration, $\alpha$. Po was computed for the right and left foot as the mean of minimums of the medio-lateral angular speed between late stance phase and pre-swing phase multiplied by the cosinus of the medio-lateral angle at that time and a constant value. Po is reported here as the minimum for the right and left foot (Figure 2).

For the preliminary study, Po was also computed using the gold standard (the force plates) with Euler's equation at the center of mass. Using the same annotations and $\overrightarrow{GRF}$, the ground force, we have:

$$\overrightarrow{((r_F - r_P)} \wedge \vec{F}).\overrightarrow{e_y} = r_P.GRF$$

which gives:
$$\frac{1}{m}.C(\vec{F}) = \frac{1}{m}.(\overrightarrow{r_F} \wedge \vec{F}).\overrightarrow{e_y} = \frac{r_F}{r_F - r_P}.\,r_P.\frac{1}{m}.GRF$$
($\overrightarrow{r_P}$ and $\overrightarrow{r_F}$ being colinear).

Thus, Po can be computed by using the gold standard as:

$$\text{Po} = \dot\alpha.C(\vec{F})/m = \frac{r_F}{r_F - r_P}.\,r_P.\frac{1}{m}.GRF.\omega \quad (\text{eq. 2}).$$

Distances between centers of the foot ($r_F$, $r_P$, $r_A$) were approximated by using reference biomechanichal standards [15], [16]:
$r_F$ = 0.2 m, $r_P$ = 0.08 m, $r_A$ = 0.2 m.

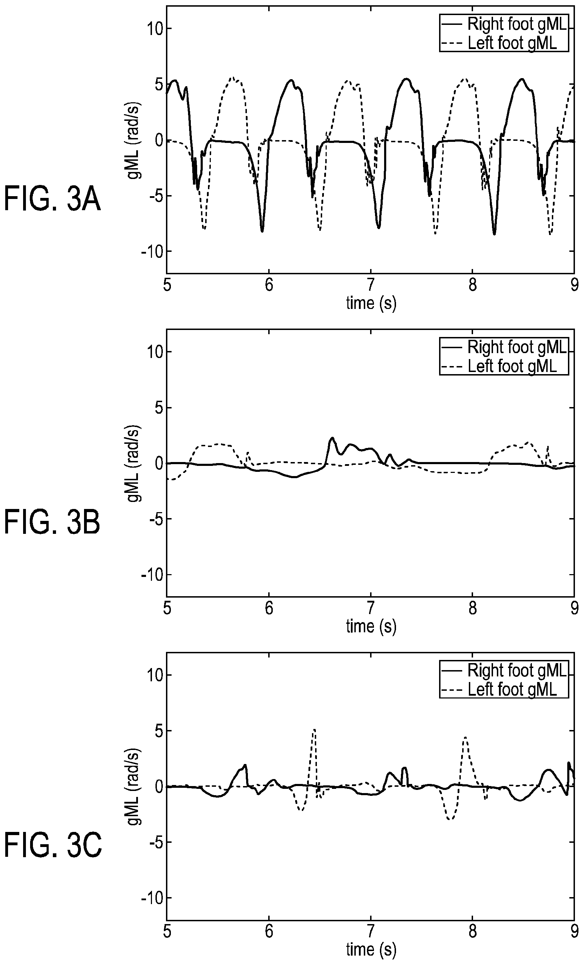

Figure 3 shows part of the signal for one HS participant with high Po (Figure 3A) and two pMS patients with decreased Po. The first pMS participant has symmetrical right-left signals (Figure 3B) and the second pMS participant shows pronounced differences between right and left (Figure 3C).
*U-turn detection* - The walk was segmented as way-in, U-turn and way-out adapting a previously published method (Barrois et al., 2017). This U-turn detection algorithm relies on the angular velocities around the cranio-caudal axis obtained from the lower back IMU. The signal was integrated to a signal giving the angular position around the cranio-caudal axis (anCC), a linear drift correction being applied during the U-turn by assuming 0° at the beginning of the turn and 180° at completion. An empirical threshold of 10° for the change in anCC during the stance phase of a step was used to detect steps belonging to the U-turn.
*Steps detection* - Initial Contacts (IC) and Final Contacts (FC) of the foot with the floor were detected manually by one assessor who relied on the description of step events by Mariani et al (Mariani et al., 2012) and trained on steps detected by an electronic pressure walkway (GaitRite®, CIR Systems, Inc., 120 Hz sample frequency), used as a validated gold standard in patients with multiple sclerosis. For this training, patients came back during their 12 months follow-up to perform a calibration gait measurement. This measure was constituted of two gait trials (6m with U-turn and back) performed on the instrumented walkway, which was synchronized to the IMUs by using the PC clock connected to these latter. The recordings from the instrumented mat were used to extract the exact timings for ICs and FCs, using the automatic algorithm embedded in its software. The assessor learnt from positions of ICs and FCs on the IMUs signals to subsequently detect them on the trials of interest. Patients were anonymized before manual processing, so that the assessor was blinded to the identity of the patient, including the group he belonged to, his characteristics (e.g. his age, weight and height, BMI) and the severity of his disease.

*Other gait kinematic parameters* - The walk was manually segmented as way-in, U-turn and way-out. Velocity (V) and 3 classic gait quality parameters [step length (SteL), stride time (StrT) and double stance time (dstT)] were computed. V was computed as the mean of the way-in and way-out V, defined as the length of the one-way (10 m) divided by the total time of the one-way path. For the other parameters, steps were detected manually. SteL was computed as the mean of the way-in and way-out SteL, defined as the length of the one-way path (10 m) divided by the total number of steps in the one-way path. For the following 3 parameters, the 2 first steps and 2 last steps of the way-in and way-out paths as well as steps from the U-turn were excluded from the analysis. StrT was defined as the time between 2 successive heel-strikes of the same foot. It is also reported as the mean of all strides (without distinguishing between the right and left foot). dstT was defined as the time between heel-strike for one foot and toe-off for the contralateral foot. dstT was reported as the mean for all steps (without distinguishing between the right and left foot). For all these parameters and for the 25FWT, Z-scores were computed on the basis of the means and SDs for HS participants. For Po for instance, the Z-score (PoZ) for patient I was computed as:

$$PoZ[i] = \frac{Po[i] - \overline{Po}}{sd(Po)}$$

where $\overline{Po}$ is the mean of Po for the 20 HS participants and *sd*(*Po*) is the standard deviation of Po for the 20 HS participants.

*Statistical analysis* - For the biomechanical study (test of Po measurement against the gold standard) and for the validation part of the clinical study, a test-retest design was chosen to evaluate the stability of the measurement between evaluations. Relative reliability was computed by using ICC(1,1) and ICC(3,1), 2 different models of intraclass correlation coefficient (ICC) and both suggested as measures of relative reliability of single measurements. In ICC(1,1), all within-subject variability is assumed as measurement error, whereas ICC(3,1) assumes the effect of any systematic bias not part of measurement error. To rule out any heteroscedasticy (in statistics, when the SDs of a variable, monitored over a specific amount of time, are non-constant) in data that would lead to a misleading ICC, Pearson's correlation coefficient (r) between the absolute differences and the individual mean values was calculated and tested against the null-hypothesis. Absolute reliability, which can be used to distinguish low ICC caused by variability within subjects from low ICC caused by narrow ranges of values within the sample, was computed with the standard error of measurement (SEM). All analyzed parameters were tested for normality by Shapiro-Wilks test. Parametric kinematic parameters (Po, V, SteL and dstT) were tested for differences between subgroups by 3-factor ANOVA with post-hoc pairwise comparisons when findings with the ANOVA model were significant. Non-parametric kinematic parameters (strT, 25FWT) were tested by Kruskall Wallis test with post-hoc pairwise comparisons (Mann-Whitney U Test). A significance threshold for post-hoc pairwise comparisons was adapted to follow Bonferroni corrections for multiple comparisons. Univariate logistic regression was used to compute odd ratios (ORs) for Po and other kinematic parameters of gait quality (SteL, StrT, dstT), estimating 95% confidence intervals (CIs). Pearson correlation coefficients for any pair of those parameters with significant ORs were computed to check for collinearity. Variance inflation factors (VIFs) were computed for all variables. When the VIF was < 10, variables were then included in a multivariate logistic regression analysis. Because Po is a continuous variable, calibration of the model was quantified by the Hosmer-Lemeshow goodness-of-fit test.

Data for M0 and M6 were included in the analysis of both Po and 25FWT. We performed Monte Carlo cross-validation nested by 4-fold cross-validation, stratified on the patient. Two thirds (10/16) of the patients were included in the training cohort and the remaining one third (6/16) in the test, or validation, cohort. Discrimination was assessed in the test cohort by estimating negative and positive predictive values, sensitivity and specificity, and the area under the receiver operating characteristic curve (AUC). The best cut-off value was determined by 2 different methods. Following the recommendations by Perkins and Schisterman [5], we used the Youden index (Y) [6], the cut-point that optimizes the test's differentiating ability when equal weight is given to sensitivity and specificity. To bias the choice of cut-off toward a higher negative predictive value, we also defined a conditional Y (Yc), the Y with the constraint that sensitivity

$$Yc = \max_{sensitivity > 90} (sensitivity + specificity - 1).$$

must be > 90% (ie, Sensitivities, specificities, Y and the AUC were computed on n = 1000 configurations of the training and test cohorts and are reported as means with corresponding 95% CIs. Negative and positive predictive values were computed with the average ROC curves by using the mean sensitivities and specificities as well as the prevalence of falls in the full dataset. Y and AUC values for Po and the 25FWT test were compared by Welch two-sample t-test (for unequal variances) with P<0.05 considered statistically significant. All statistical analyses involved using R v3.5.1.

**Results**

*1. Biomechanical study: Po is a reliable surrogate of ankle push-off moment*

We included 7 healthy subjects (4 female; mean age 25.5 [range 21-29 years]) in the biomechanical study. The mean height was 167.3 (range 150-182) cm, mean weight 58.4 (range 42-70) kg and mean body mass index 20.5 (range 17.0-23.9) kg/m2. Data heteroscedasticity was ruled out for computation of Po both for individual steps (right foot: r = -0.10, p = 0.55; left foot: r = -0.03, p = 0.74) and the total walk (right foot: r = 0.15, p = 0.75; left foot: r = 0.28, p = 0.55; minimum of right and left foot: r = 0.00, p = 0.99).
Comparison of Po measured with IMUs (eq. 1) and with force plates (eq. 2) showed good ICC values for both individual steps (right foot: ICC(1,1) = 0.76, ICC(3,1) = 0.76; left foot: ICC(1,1) = 0.79, ICC(3,1) = 0.79, Figure 5A) and the total walk (right foot: ICC(1,1) = 0.49, ICC(3,1) = 0.83; left foot: ICC(1,1) = 0.76, ICC(3,1) = 0.76; minimum of right and left foot: ICC(1,1) = 0.74, ICC(3,1) = 0.74, Figure 5A).
For this test-retest, the SEM of Po for individual steps was 0.15 W/kg for the right Po and 0.14 W/kg for the left Po (Figure 5B). The SEM of Po for the total walk was 0.05 W/kg (Figure 5B).

*2. Validation study: one-time measurement of Po can be used as a screening tool for falls*

*Demographic characteristics of the populations included*

Sixteen patients were included in the pMS training cohort (Figure 4). One patient was lost to follow-up at 6 months because he stopped visits to his neurologist. Data from this patient were included until loss of follow-up (M0 measures and number of falls between M0 and M6). The baseline characteristics of the 16 patients are in Table 1. Half were female and the mean age at M0 was 54.8 ± 8.2 years. A total of 12 patients (75%) had secondary progressive disease and 4 (25%) had primary progressive disease. In the entire group of patients, the mean time from MS onset to inclusion was 13.6 ± 8.2 years. Overall, 10 patients (63%) underwent physical therapy in the previous 6 months, with a mean time of 1.5 ± (2.6) hr per week. In total, 8/16 (50%) experienced a fall during the first 6-month period (between M0 and M6) and 8/15 (53%) during the second 6-month period (between M6 and M12). Among the patients who fell during the first 6-month period, 1 from the pMS group stopped falling during the second 6-month period. Among the patients who did not fall during the first 6-month period, 1 patient began falling during the second 6-month period.

*Po is a reproducible and repeatable parameter for HS participants and pMS patients*

For both pMS patients and HS participants, data heteroscedasticity was ruled out for the CG versus UG test-retest (MS: r = -0.13, p = 0.63; HS: r = -0.34, p = 0.16) and for the M6 versus M0 test-retest (MS: r = -0.02, p = 0.95; HS: r = -0.02, p = 0.94). For pMS patients, test-retest agreements were high both for the CG versus UG condition (ICC(1,1) = 0.99 and ICC(3,1) = 0.99) and M6 versus M0 (ICC(1,1) = 0.96 and ICC(3,1) = 0.96), which indicates agreement from a relative perspective. HS participants showed lower but still high test-retest agreements for the CG versus UG condition (ICC(1,1) = 0.82 and ICC(3,1) = 0.82) and medium test-retest agreements for M6 versus M0 (ICC(1,1) = 0.61 and ICC(3,1) = 0.61) (Figure 6A). For the CG versus UG test-retest, the SEM for Po was 0.02 and 0.03 W/kg for pMS and HS participants, respectively. For the M6 versus M0 test-retest, the SEM for Po was -0.53 and 0.07 W/kg for pMS and HS participants, respectively (Figure 6B).

*Po at M0 differentiates fallers from non-fallers and predicts falls within 6 months in progressive MS*

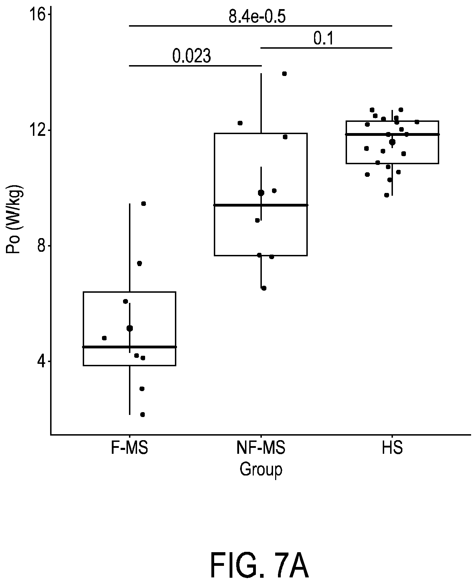

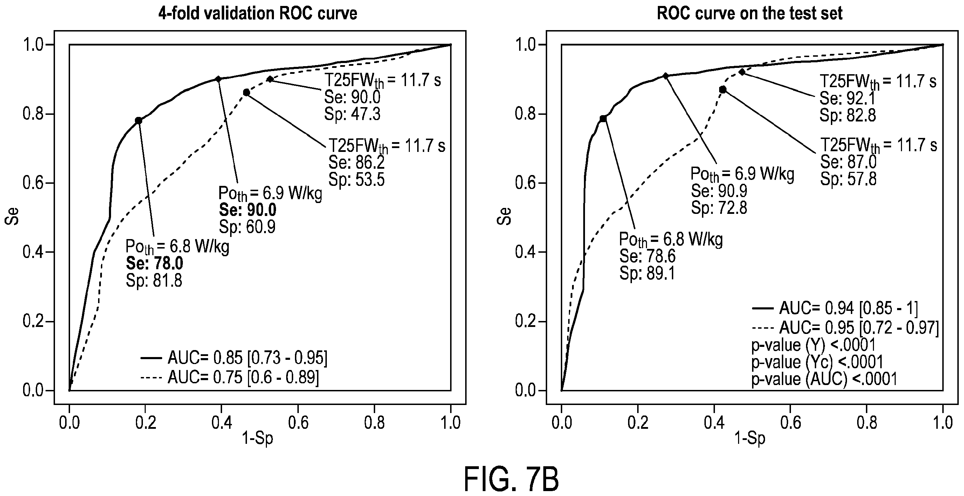

Mean Po was lower for pMS participants with falls (pMS-F) than non-fallers (pMS-NF) (at M0: 5.2 vs 9.6 W/kg, unpaired t-test: p=0.002, 95% CI for mean difference -7.0-1.8) and HS participants (5.2 vs 11.6 W/kg, unpaired t-test: p<.0001, 95% CI for mean difference -8.3--4.3). Po was decreased for pMS-NFs versus HS participants but not significantly (unpaired t-test: p-value = 0.10, 95% CI for mean difference -4.0-0.2) (Figure 7A and Table 2). In the pMS training cohort (n=10) (Figure 7B), we identified a Po cutoff of 6.8 W/kg as having the most important Y (accuracy) and a Po cutoff of 6.9 W/kg as having the most important cY (accuracy with constraint on sensitivity to bias the test toward screening). In a validation study, among the pMS testing cohort (n=6) (Figure 7B), a Po of ≤ 6.9 W/kg had a negative predictive value (i.e., no falls in the subsequent 6 months) of 83.7% (95% CI 83.1-84.4%), with 90.9% sensitivity (95% CI 90.0-91.7%) and 72.8% specificity (95% CI 71.1-74.4%) The AUC with the testing cohort was 0.94 (95% CI 0.85-1.00).

*Po shows better classification accuracy than the 25FWT*

In our cohort, the best cutoff value for the gold standard, the 25WFT, was 11.7 s with both the Y and cY (non-significant difference based on number of digits). The validation study with the testing pMS cohort (n=11) with a value of $\geq$ 11.7 s had a negative predictive value (i.e., at least one fall in the subsequent 6 months) of 82.9% (95% CI 82.0-83.7%), with 92.1% sensitivity (95% CI 91.0-93.2) and 52.8% specificity (95% CI 50.9-54.6). The AUC was 0.85 (95% CI 0.72-0.97). ROC curves for both the Po and 25WFT in the test cohort in Figure 7B, showing a significant difference between both evaluations in terms of AUC (0.94 vs 0.85, Welch t-test: p-value: <.0001), Y (0.60 vs 0.40, Welch t-test: p-value: <.0001) and cY (0.51 vs 0.37, Welch t-test: p-value: <.0001).

*One-point increase in Po Z-score reduces the risk of falls in pMS by half and its evolution predicts a change in fall risk*

On univariate analysis, in the pMS group, a 1-W/kg increase in Po could reduce the risk of reporting a fall in the following 6 months by half (ORraw = 0.56, 95% CI 0.36-0.76) (Table 3). To relate these findings with risks associated with other kinematic parameters, their ORs based on Z-scores were compared. The univariate OR for Z-score of Po was 0.60 (95% CI 0.40-0.78) versus 0.89 for Z-score of 25FWT (95% CI 0.76-0.98) and 0.48 for Z-score of V (95% CI 0.27-0.74]). The univariate ORs for Z-scores of SteL, dstT and strT were 0.67 (95% CI 0.46-0.89), 0.62 (0.37-0.91) and 0.81 (0.68-0.92) (Table 3).

All of these quality parameters were significantly associated with falls and thus included in the multivariate analysis. Because these parameters were highly correlated in pMS patients (supplemental Figure 2), VIFs were computed and were < 10 for all parameters (PoZ: 7.7, dstTZ: 3.8, SteLZ: 5.7, strTZ: 6.5). Only Po remained predictive of falls in this multivariate analysis, with an adjusted OR of Z-scores of 0.31 (95% CI 0.09-0.65) (Table 4). Of note, 3 patients from the pMS cohort decreased their Po by more than 20%. All showed increased or the same number of falls in the following 6 months (Figure 8).

## Discussion

This study proposes a method to calculate an estimate of the push-off, Po, by using IMUs. We also show that Po allows for predicting falls in people with pMS as well as between-visit comparisons and characterization of treatment-induced effects. The technique can be used in routine neurological practice to assess gait quality within the time constraint of a visit and without the need for dedicated space, contrary to what is currently needed when using force plates.

In the preliminary biomechanical analysis in healthy subjects, Po showed good internal reliability as well as good external validity because we found values comparable to what was published in the literature, ranging from 8 to 15 W/kg. The screening test with Po can be performed within the time constraints of current patient intake processes and requires unintrusive, cheap and light equipment. The analysis was done manually, but computerized step detection and analyses are being developed and are becoming widely available.

As compared with other usual kinematic parameters, Po presents 3 key advantages for use in screening for fall risk: first, it is a direct indicator for targeted therapy or symptomatic treatment (foot orthoses). Second, is it less likely an adaptative reaction to fall risk as reducing speed can be. Also, Po is a reliable parameter, robust to instructions regarding eye fixation, which can be considered as an additional cognitive load or a help for straight walking. These features are important for clinical practice in which tests are not performed under similar conditions. For instance, corridors can be busy during some office visits and empty during others. Third, Po is also repeatable at 6 months, which usually corresponds to the next follow-up visit with the clinician. The SEM of Po for the M6 versus M0 measurement was 0.53 W/kg for pMS patients, which could be considered the smallest change threshold that indicates a change.

Several studies used video motion analysis to analyse ankle kinematic during late-stance in pMS and reported reduced maximum ankle plantarflexion and total angular excursion in pMS, with EDSS scores ranging from 0 to 4. Using inverse dynamics from video motion analysis data (Vicon®), Huisinga et al. [7] computed ankle power at toe-off and found a 23% decrease in Po values in pMS with EDSS score 1-4 as compared with healthy participants. In agreement, we found reduced Po in people with pMS. Still, it should be noted that the values for the latter study were much lower than those we report (3.1 $\pm$ 0.9 W/kg for HS and 2.4 $\pm$ 0.7 W/kg for pMS in the Huisinga et al. study [7]), which indicates the high heterogeneity regarding absolute values for joint ankle power even in distinct studies using the same apparatus (video motion analysis) [7]-[9].

This altered push-off, which indicates decreased active muscle concentric contraction or return of the energy stored in soft tissues, is thought to be mainly due to lower-extremity weakness and spasticity, which is responsible for reduced and desynchronized activation of extensor muscles during late-stance. Consequences on the gait pattern include reduced velocity, cadence and step length, altered stance/swing phase ratio and increased double support time. Unsurprisingly then, Po was strongly associated with alterations in speed, step length and ratio of double support time to stride time and, to a lesser extent, decreased cadence. Of note, Po was the only parameter that predicted falls in a multivariate analysis including those other parameters. As well, in our cohort, Po was not significantly decreased in non-fallers with pMS versus HS participants, in contrast to the other kinematic parameters. Although the difference might reach significance in a larger cohort, this difference of effect with the other kinematic parameters might be explained by a higher specificity of Po as a predictor of falls than these other parameters.

Use of IMUs in clinical practice is becoming an increasingly informative tool to understand disease evolution. Deficits in the push-off moment could predict falls in our pMS patients, and a patient with Po ≤ 6.9 W/kg had a 9 in 10 probability of falling in the following 6 months. In addition, decreased Po between 2 evaluations at a 6-month interval should be considered a red flag for risk of falling. Consequently, Po can be used both as a follow-up biomarker in clinical practice for pMS patients and a direct indicator for targeted physical therapy.

Tables

Table 1: Baseline characteristics of patients with progressive multiple sclerosis (pMS) and healthy subjects (HS).

| | pMS (n=16) | | HS (n=20) | |
|---|---|---|---|---|
| | mean | SD | mean | SD |
| Sex (M/F) | 8/8 | - | 10/10 | - |
| Age (years) | 54.8 | 8.2 | 59.4 | 13.1 |
| Years since diagnosis | 13.6 | 8.0 | - | - |
| Years since first sign | 14.8 | 8.7 | - | - |
| Years since progression | 8.9 | 7.7 | - | - |
| EDSS | 5.0 | 1.4 | - | - |
| Pyramidal function | 2.9 | 1.2 | - | - |
| Cerebellar function | 1.8 | 1.3 | - | - |
| Bulbar function | 0.9 | 1.2 | - | - |
| Sensitive function | 0.9 | 1.1 | - | - |
| Uro-digestive function | 1.7 | 1.7 | - | - |
| Displaying function | 0.2 | 0.6 | - | - |
| Cognitive function | 1.6 | 1.4 | - | - |
| CSCT | -1.3 | 1.6 | -0.7 | 0.9 |
| Total answers | 35.1 | 16.1 | 41.5 | 8.1 |
| Total good answers | 35.8 | 15.8 | 40.5 | 7.3 |
| MSWS | 75.8 | 15.6 | - | - |
| FIS | 50.6 | 24.0 | - | - |
| Cognitive | 38.1 | 34.6 | - | - |
| Physical | 68.6 | 22.2 | - | - |
| Social | 41.5 | 25.7 | - | - |
| Psychological | 54.2 | 31.0 | - | - |
| Physical therapy in the past 6 months (yes/no) | 10 / 6 | - | 0 / 20 | - |
| Use of walking aid for the walk test (yes/no) | 8 / 10 | - | 0 / 20 | - |
| Cane (1 or 2) | 5 | - | - | - |
| Walker | 2 | - | - | - |
| Human help | 1 | - | - | - |
| Cane + human help | 0 | - | - | - |

EDSS, Expanded Disability Status Scale; CSCT, Computerized Speed Cognitive Test; MSWS, Multiple Sclerosis Walking Scale-12; FIS, Fatigue Impact Scale

**Table 2:** Kinematic values in the pMS cohort with and without falls (pMS-F and pMS-NF) and HS cohort.

| Parameters | pMS-F (n=8) | pMS-NF (n=8) | HS (n=19) | p-value pMS-F vs pMS-NF | p-value pMS-F vs HS | p-value pMS-NF vs HS |
|---|---|---|---|---|---|---|
| Po (W/kg) | 5.2 [3.2–7.2] *# | 9.6 [7.8–11.3] | 11.6 [11.2–12.0] | 0.002 | < .0001 | 0.10 |
| 25FWT (s) | 17.0 [11.7–22.3] *# | 11.6 [7.06–16.1] | 6.9 [6.1–7.4] | 0.020 | 0.003 | 0.060 |
| V (m/s) | 0.51 [0.31–0.70] *# | 0.89 [0.69–1.09] † | 1.14 [1.07–1.21] | 0.021 | < .0001 | 0.015 |
| SteL (m) | 0.40 [0.25–0.56] # | 0.55 [0.45–0.65] | 0.67 [0.63–0.70] | 0.200 | 0.004 | 0.079 |
| StrT (s) | 1.55 [1.25–1.85] # | 1.29 [1.04–1.53] | 1.13 [1.09–1.17] | 0.023 | 0.011 | 0.14 |
| dstT (% stride) | 31.3 [25.8 – 36.8] # | 27.6 [18.6 –36.6] † | 16.2 [13.7–18.6] | 0.42 | 0.0001 | 0.019 |

Results are reported as mean [95% confidence intervals (CIs)].
Pairwise comparisons were performed, with significant results stated as * (pMS-F vs pMS-NF), # (pMS-F vs HS) and † (pMS-NF vs HS).
25FWT, 25-foot walk test; V, velocity; Stel, step length; StrT, stride time; dstT, double stance time;

**Table 3:** Univariate analysis of parameters and parameter Z-scores in patients with pMS (n = 16).

| Parameters | ORraw | ORZscore |
|---|---|---|
| Po (W/kg) | 0.56 [0.36-0.76] | 0.60 [0.40-0.78] (1 SD = 0.86 W/kg) |
| SteL (m) | 2.51 e-03 [1.06e-05-0.18] | 0.67 [0.46-0.89] (1 SD = 0.08 m) |
| StrT (s)-decrease | 0.073 [0.01- 0.37] | 0.81 [0.68- 0.92] (1 SD = 0.07 s) |

(continued)

| Parameters | ORraw | ORZscore |
|---|---|---|
| dstT (% stride)-decrease | 0.91 [0.83-0.98] | 0.62 [0.37-0.91] (1 SD = 4.00%) |
| 25FWT (s)-decrease | 0.90 [0.78-0.98] | 0.89 [0.76-0.98] (1 SD = 0.50 s) |
| V (m/s) | 9.46e-03 [2.12e-04-0.1] | 0.48 [0.27-0.74] (1 SD = 0.18 m/s) |

Data are odds ratios (ORs) and 95% CIs.

**Table 4:** Multivariate analysis of parameters and parameter Z-scores in patients with pMS with unchanged treatment (MS cohort) (n = 16).

| | ORraw | ORZscore |
|---|---|---|
| Po (W/kg) | 0.27 [0.07-0.62] | 0.31 [0.09-0.65] (1 SD = 0.86 W/kg) |
| SteL (m) | 1.2e+04 [0.03-1.89e+11] | 1.89 [0.80-5.84] (1 SD = 0.08 m) |
| StrT (s)-decrease | 6.92e-03 [1.20e-05-1.11] | 1.37 [0.97-2.15] (1 SD = 0.07 s) |
| dstT (% stride)-decrease | 0.95 [0780-1.14] | 0.74 [0.26-2.03] (1 SD = 4.00 %) |

Data are ORs [95% CIs].

STUDY OF ESTIMATION OF PUSH-OFF DEFICIT TO FOLLOW-UP PATIENTS UNDER HIGH-DOSE BIOTIN TREATMENT

**METHODS**

We perform a prospective observational study to evaluate a measure of a surrogate of ankle push-off moment power (Po) as a biomarker of evolution in pMS patients with stable treatment and pMS patients with newly introduced MD1003 (high-dose biotin). Po is measured during a 10m-walk with U-turn and back, using an IMU attached to each foot. The objective is to assess whether the dynamic measure of Po at 6-month interval reflects the reponse to treatment (assessed using the change in the timed 25-foot walk or the Expanded Disease Status Scale) and the change in fall risk within 6 months.

**RESULTS**

The evolution of Po has been analyzed in 17 patients where MD1003 is introduced (pMS-b), and in 16 patients without recent change in treatment (pMS-nb) as well as a group of 20 age and sex-matched healthy subjects (HS). Two patients from the pMS-nb cohorts dropped out before M6. All responders (0/14 pMS-nb and 2/17 pMS-b) show increased Po between M0 and M6 and a decreased or equal number of falls in a 6-month follow-up. Patients show increased risk of falls at M6 compared to M0 only when Po is also significantly altered (≥20% decrease) at M6 as compared to M0.

**CONCLUSIONS**

Po seems to be a valid parameter for longitudinal follow-up of pMS to evaluate response to treatment and screen for fall risk. A previously validated Po threshold of 6.9 W/kg or lower can be used to predict fall risk within 6 months in pMS treated with MD1003.

**Introduction of the study**

The Timed 25-foot Walk Test (T25FW) is the gold standard to evaluate the evolution of gait impairment in pMS, and has been used for evaluation of treatment effect. Indeed, gait speed is a valuable index [metaMS]. Neverthless, its evaluation using the T25FW has been criticized for being highly variable and its relevance for the patient quality of life has still to be appraised. Besides, decreased velocity is not pathological per se but is the result of underlying phenomena causing decreased step length or cadence, and can sometimes be regarded as a useful adaptative process. New biomarkers of gait impairments in pMS would be therefore worth exploring.

A potential candidate is the ankle 'push-off', which is the peak of the shortening contractions of the plantar flexor muscles - the soleus and the gastrocnemius - during the late-stance. It is also decreased in patients with pMS. Previous studies associated alteration of this push-off with falls as this deficit was held responsible for reduced toe clearance during the initial swing which favorizes tripping. Moreover, preserved push-off of the support limb during tripping can help recovery by providing time and clearance for adequate positioning of the swinging foot and by restraining the angular momentum of the body during push-off. Thus, when the push-off decreases, pMS patients are more likely to sustain a fall rather than a near-fall when transferring outside the home and tripping over an obstacle. It remains that motion analysis system and/or force platform are required to quantify the push off, which is not convenient for routine medical visits.

The inventors tested pMS patients treated with MD1003, an oral formulation of high-dose biotin which recently demonstrated encouraging efficacy in decreasing the T25FW time by 20% in 10%.

A first goal in this paper is to test whether Po can reflect change - and absence of change - in patients treated with MD1003.

Second, the inventors aim at measuring how this variation in Po impact evolution of fall risk in this group of pMS patients. They performed a 12-month prospective analysis of 33 pMS patients from which half were treated with MD1003 (pMS-b) and the other half was set as a control group of patients who did not receive MD1003 (pMS-nb). The T25FW, Po and risk of falls was assessed every 6 months using a simple validated protocol and the evolution of Po and the risk of falls were compared for each individual according to his response to treatment.

**Methods:**

*Patients* -Thirty-three patients with progressive MS (pMS) and 20 gender and age-matched healthy subjects (HS) were enrolled in this longitudinal prospective study (table 1). pMS were recruited consecutively from the outpatient clinic of Percy Hospital (Clamart, France) between December 2017 and April 2018. The inclusion criteria for participation in the pMS cohorts required participants to be at least 18 years old, be diagnosed with primary progressive or secondary progressive multiple sclerosis according to the 2010 International Panel criteria [10], be capable of walking 20m with U-turn, be free of any other conditions that affect gait. The exclusion criteria was pregnancy and high-dose biotin intake before M0. From the pMS cohort, the inventors isolated a subgroup, named pMS-b cohort, built with patients who were treated with high-dose biotin (MD1003 100mg, three times a day) for at least one year after inclusion. For participating in the pMS-b cohort, additional inclusion criteria were those of the cohort Temporary Use Authorisation (TUAc) of MD1003 - they had to have been free of any relapse for at least one year and sign the consent to enter the TUA cohort. The other patients were included in a group named pMS-nb. Exclusion criteria from this pMS-nb cohort were the presence of drug intake modification for the six-months prior to inclusions or during the twelve-month follow-up. HS participants were recruited from the hospital and research unit staff between December 2017 and April 2018. The inclusion criteria for participation in the HS cohort included: no report of falls in the past 5 years before inclusion, no disease that could affect their walk. Seventeen patients were included in the pMS-b cohort and 16 in the pMS-nb cohort. All participants gave their written consent to participate in the study. The study protocol was conducted according to the Helsinki principles and approved by Ethic Comity "Protection des Personnes Nord Ouest III" under the ID RCB : 2017-A01538-45.

*Measures* - Gait was measured using four 3-dimensional accelerometers (Mtw XSens®, 100Hz sampling frequency) positioned on the head, lower back (L4-L5 vertebrae) and dorsal part of both feet. Participants performed two walks of 20m with U-turn (10m way in and 10m way out). One trial per visit only was used in this study, as the second trial was done with specific conditions for the sake of a reproducibility test in a previous study. A limit at 7.62m was drawn for the assessor to timeclock the T25FW durint the first way-in. Disease severity was assessed using patient-reported outcomes (the Multiple Sclerosis Walking Scale-12 - MSWS - and the Fatigue Impact Scale - FIS) together with clinical evaluation (Expanded Disease Severity Scale [10] - EDSS - and Computerized Speed Cognitive Test [11] - CSCT) and the 25-foot walking test administered according to a validated standardized protocol [12]. At first visit (M0), participants were asked for the number and circumstances of falls in the previous six months, with falls being described as "an event which results in a person coming to rest unintentionally on the ground or other lower level, not as a result of a major intrinsic event (such as a stroke) or overwhelming hazard" [13]. They were asked to record and note down any fall that would happen. They were seen at three (M3), six (M6) and twelve months (M12) after the initial visit to evaluate the number of falls they had had since this visit. Participants who fell within 6 months following a visit were reported as fallers at that visit (F-pMS for pMS or F-HS for HS) whereas patients who did not fall were called non-fallers (NF-pMS for pMS or NF-HS for HS). Patients from the pMS-b cohort had their M0 visit in the week before MD1003 initiation. MD1003 was maintained over 12 months if the patient displayed a 20% improvement at the T25FW or an improvement in EDSS ($\geq$1

point decrease if the initial score is between 4.5 and 5.5 or ≥0.5 point if the initial score is between 6.0 and 7.0) as defined in the TUA guidelines. Patients from the pMS-nb cohort did not receive biotin during the 12-month follow-up. ***Definition of groups according to their response to treatment*** - In each of the three cohorts (MS-b, MS-nb, HS), five groups were defined according to their change in T25FW between M0 and M6. "Complete responders" were defined as participants who decrease their T25FW at M6 by ≥20% as compared to M0. "Partial responders" had a T25FW decrease of 10 to 20% at M6 compared to M0. "Non responders" had a T25FW change of -10 to 10% at M6 compared to M0. "Partial Progressors" increase their T25FW at M6 by 10 to 20% as compared to M0. "Complete Progressors" increase their T25FW at M6 by ≥20% as compared to M0.

*Kinematic parameters estimation* - Velocity (V) was computed as the mean of way-in and way-out velocity, defined as the length of the one-way (10m) divided by the total time of the one-way. As defined in the present patent application, a surrogate of the moment power per kilogram released by the ankle push-off moment (Po) was defined as the product of the torque of muscle force per kilogram in the sagital plane ($C(\overrightarrow{F})$) and the joint angular velocity ($\omega = \dot{\alpha}$, with $\dot{\alpha}$ being the angle between the floor and the plantar line) divided by the body mass (m):

$$\mathrm{Po} = \frac{1}{m} . C(\overrightarrow{F}) \times \omega.$$

Both torque and joint angular velocity can be derived using IMUs.

In a similar manner to the threshold of significance for the T25FW, an increase of ≥20% in Po was considered significant.

**Results**

*1. Demographic characteristics of the populations included*

Seventeen patients were included in the pMS-b cohort and 16 in the pMS-nb cohort. Two patients from the pMS-nb group were lost for follow-up at 6-month because they interrupted their visits to their respective neurologist. Data from both patients were included until loss of follow-up (M0 measures and number of falls between M0 and M6). The base-line characteristics of the 33 patients included are given in Table 1. Fifty percent of patients were female and the mean age at M0 was 58.2 ± 10.5 years. A total of 23 patients (70%) had secondary progressive disease (SP patients), whereas 10 patients (30%) had primary progressive disease (PP patients) at diagnostic. In the entire group of patients, the median time from the onset of multiple sclerosis to inclusion was 17.6 ± 10.7 years. Sixty-seven percent underwent physical therapy in the six previous months with a mean time of 2.1 ± 2.6 hours per week. Of all pMS included, 16/33 (48.5%) experienced a fall during the first 6-month period (between M0 and M6) and 16/31 (51.7%) experienced a fall during the second 6-month period (between M6 and M12). From the patients who fell during the first 6-month period, 1 patient from the pMS-b group (EDSS of 6.0) and 1 patient from the pMS-nb group (EDSS of 4.0) stopped falling during the second 6-month period. From the patients who did not fall during the first 6-month period, 3 from the pMS-b group (EDSS of 3.5, 3.5 and 6.0) and 1 from the pMS-nb group (EDSS of 5.5) began falling during the second 6-month period. In the pMS-b cohort, 3/17 patients (18%) were complete responders (EDSS of 4.0, 5.5 and 6.0), while there was no responder in the pMS-nb cohort.

*2. Po varies with treatment that alters gait and its evolution can be used to predict the evolution of fall risk*

*The measure of Po at M0 gives good fall predictions but not at M6*

The inventors analyzed the evolution of Po in the pMS-b group as compared to its evolution in the pMS-nb and HS groups (Table 2). A cut-off of 6.9 W/kg was shown as having good predictive value of falls in the following 6-month period in the pMS-nb group. Indeed, screening for subsequent falls within a 6-month period at M0 in the pMS-nb cohort with a cut-off of 6.9 W/kg gives a sensitivity and a specificity of 75.0 and 87.5 respectively (1 false positive and 2 false negatives). Measuring Po at M6 for the prediction of falls between M6 and M12 with a cut-off of 6.9 W/kg gives a sensitivity and a specificity of 100.0 and 85.7 respectively (1 false positive and no false negative). The same measure at M0 in the pMS-b cohort with a cut-off of 6.9 W/kg gives a sensitivity and a specificity of 88.9 and 75.0 respectively (2 false positives and 1 false negative). At M6 in the pMS-b cohort the sensitivity and a specificity are 54.5 and 50.0 respectively (3 false positives and 5 false negatives).

*Significant increase in Po is more frequent in the pMS-b group than in the pMS-nb and HS groups*

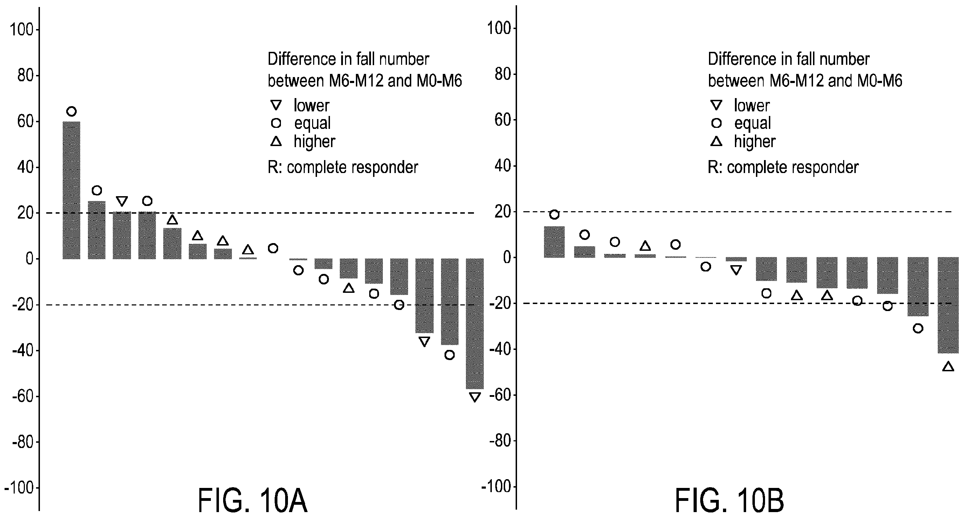

In the pMS-b group, 8 out of the 17 patients increased their Po, with 4 showing an increase of more than 20% (Figure 10A). From these 4 patients, 3 were complete responders (≥20% decrease in T25FW) and one showed partial response (decrease in T25FW of 14%). In the pMS-nb group, 5 out of the 14 patients increased their Po but only one patient increased his Po of more than 20% (Figure 10B). No HS participants showed an increase in Po higher than 20% or lower than -20% (range of change in Po in HS participants: -19% to 10%).

*A significant increase in Po between two evaluations predicts fall risk decrase or stability*

In the pMS-b group, the 4 patients who showed a significant increase (higher than 20% change) in Po reported an equal or lower number of falls in the following 6 months (Figure 10A). Three participants from the pMS-b cohort and 3 from the pMS-nb cohort decreased their Po by more than 20%. From these patients, all participants from the pMS-nb cohort increased or kept the same number of falls in the six following months. Two of these three participants from the pMS-b cohort however decreased their number of falls, while the other kept falling as often (5-6 falls per week) (Figure 10A). Comparing the variation of Po to the variation of gait speed (Figure 10B), it can be seen that all patients with reduced number of falls and decreased Po (either from the pMS-b cohort or the pMS-nb cohort) had decreased their speed more than their Po.

**Discussion**

This study shows that Po permits between-visits comparisons and characterization of treatment-induced effects. Also, in our small sample of patients, MD1003 improved the locomotion in a subset pMS patients. A larger cohort should be tested to confirm that trend.

The series of pMS-b patients included in this article is comparable to the previous cohort from the phase 3 clinical trial in terms of gender (50% in our cohort versus 51.5% in [14]), EDSS (5.5 ± 1.1 in our cohort versus 6.0 ± 0.8 in [14]) and response to treatment as defined by a 20% decrease in TW25F (13% in our cohort versus 8.7% in [14]). Our cohort was slightly older (61.2 ± 11.1 in our cohort versus 51.8 ± 9.1 in [14]), with a little higher SP versus PP ratio (2.3 in our cohort versus 1.5 in [14]) and time since diagnostic (21.1 ± 11.0 in our cohort versus in 14.8 ± 8.9 years [14]).

Both the pMS-nb group and the pMS-b group had lessened Po as compared to HS participants (Table 6). Nevertheless, while the fall prediction given by Po was good for the first 6-month period of the evaluation in this group of patients, it was highly altered for the next 6-month period. MD1003 is thought to be acting mainly after 9 months taking the drug: decreased predictive performance of Po measured at M6 could then result of a change in the course of the evolution of the disease which results from the drug. As a matter of fact, no change in the predictive performance of Po was observed between the two 6-month period in the pMS-nb group. Using the sole threshold of Po should therefore be made cautiously in patients undergoing change in their treatment. Indeed, the beginning of MD1003 in the pMS-b cohort led to significant change of Po in nearly half the cohort. Interestingly however, a positive and significant evolution of Po was predictive of a decrease in fall risk between the 6-month period following M0 and the 6-month period following M6. Remarkably, a negative and significant decrease in Po could also result in diminished fall risk when the decrease in speed was higher than the decrease in Po ("cautious" zone from Figure 11). This reduction of gait speed could be regarded as a cautious adaptive process by patients trying to avoid falls. Change in Po prompts modification of walking to which the patient has to adapt. Measuring patient at M6 might then be too early to draw definitive conclusions as regards the effects of MD1003. Further analysis for a 18-month follow-up should be welcome to validate these results.

<u>**Tables**</u>

Table 5 - Baseline characteristics of patients with pMS (pMS-b and pMS-nb respectively) and controls.

| | **pMS-b** (n=17) | | **pMS-nb** (n=16) | | **HS** (n=20) | |
|---|---|---|---|---|---|---|
| | *mean* | *SD* | *mean* | *SD* | *mean* | *SD* |
| *gender (M/F)* | *8/9* | - | *8/8* | - | *10/10* | - |
| *age (yrs)* | *61,2* | *11,1* | *54,8* | *8,2* | *59,4* | *13,1* |
| nb of years since diagnostic (yrs) | 21,1 | 11,0 | 13,6 | 8,0 | - | - |
| nb of years since first sign | 27,3 | 11,6 | 14,8 | 8,7 | - | - |
| nb of years since progression | 14,8 | 9,3 | 8,9 | 7,7 | - | - |
| *EDSS* | *5,5* | *1,1* | *5.0* | *1,4* | - | - |

(continued)

| | pMS-b (n=17) | | pMS-nb (n=16) | | HS (n=20) | |
|---|---|---|---|---|---|---|
| | *mean* | *SD* | *mean* | *SD* | *mean* | *SD* |
| *pyramidal function* | *3,1* | *0,6* | *2,9* | *1,2* | *-* | *-* |
| *cerebellar function* | *1,5* | *1,3* | *1,8* | *1,3* | *-* | *-* |
| *bulbar function* | *0,4* | *0,7* | *0,9* | *1,2* | *-* | *-* |
| *sensitive function* | *1,6* | *1,4* | *0,9* | *1,1* | *-* | *-* |
| *uro-digestive function* | *2,2* | *1,8* | *1,7* | *1,7* | *-* | *-* |
| *displaying function* | *0,2* | *0,5* | *0,2* | *0,6* | *-* | *-* |
| *cognitive function* | *1,3* | *1,5* | *1,6* | *1,4* | *-* | *-* |
| ***CSCT*** | ***-1,6*** | ***1,7*** | ***-1,3*** | ***1,6*** | ***-0,7*** | ***0,9*** |
| *nb answers* | *32,1* | *16,2* | *35,1* | *16,1* | *41,5* | *8,1* |
| *nb good answers* | *32,7* | *15,8* | *35,8* | *15,8* | *40,5* | *7,3* |
| ***MSWS*** | ***69,4*** | ***24,7*** | ***75,8*** | ***15,6*** | ***-*** | ***-*** |
| ***FIS*** | ***46,5*** | ***16,9*** | ***50,6*** | ***24,0*** | ***-*** | ***-*** |
| *FIS - cognitive* | *32,4* | *24,5* | *38,1* | *34,6* | *-* | *-* |
| *FIS - physical* | *60,5* | *15,6* | *68,6* | *22,2* | *-* | *-* |
| *FIS - social* | *38,7* | *23,1* | *41,5* | *25,7* | *-* | *-* |
| *FIS - psychological* | *54,2* | *27,5* | *54,2* | *31,0* | *-* | *-* |
| ***physical therapy in the past 6 months*** *(yes/no)* | ***12 / 5*** | *-* | ***10 / 6*** | *-* | ***0 / 20*** | *-* |
| ***use of walking aid for the walk test*** *(yes/no)* | ***5 / 12*** | *-* | ***7 / 9*** | *-* | ***0 / 20*** | *-* |
| *cane (1 or 2)* | *3* | *-* | *4* | *-* | *-* | *-* |
| *walker* | *1* | *-* | *2* | *-* | *-* | *-* |
| *human help* | *0* | *-* | *1* | *-* | *-* | *-* |
| *cane + human help* | *1* | *-* | *0* | *-* | *-* | *-* |

Table 6 - Six-month follow-up for pMS-b, pMS-nb and HS patients

| | pMS-b (n=17) | pMS-nb (n=14) | HS (n=20) |
|---|---|---|---|
| **complete responder (≥i20% decrease)** | **3 (18%)** | **-** | **2 (10%)** |
| mean change in TW25F (95% CI) | -0.26 [-0.36 - -0.15] | - | -0.23 [-0.26 - -0.2] |
| mean change in Po (95% CI) | 0.33 [-0.14 - 0.81] | - | 0.03 [-0.02 - 0.07] |
| **partial responder (10-20% decrease)** | **2 (12%)** | **1 (7%)** | **3 (15%)** |
| mean change in TW25F (95% CI) | -0.17 [-0.18 - -0.16] | -0.18 [NA] | -0.11 [-0.12-0.09] |
| mean change in Po (95% CI) | 0.14 [-0.06 - 0.33] | -0.16 [NA] | -0.05 [-0.14 - 0.04] |
| **non responder (-10 - 10% decrease)** | **5 (29%)** | **8 (57%)** | **13 (65%)** |
| mean change in TW25F (95% CI) | 0.00 [-0.09 - 0.1] | 0.03 [-0.06 -0.12] | 0.00 [-0.11 - 0.11] |
| mean change in Po (95% CI) | -0.02 [-0.12 - 0.08] | -0.04 [-0.28 - 0.2] | 0.00 [-0.18 - 0.18] |
| **partial progressor (10-20% increase)** | **2 (12%)** | **2 (14%)** | **1 (5%)** |
| mean change in TW25F (95% CI) | 0.17 [0.1 -0.23] | 0.15 [0.14 - 0.16] | 0.17 [NA] |
| mean change in Po (95% CI) | 0.05 [-0.39 - 0.49] | -0.13 [-0.17 -0.09] | 0.03 [NA] |
| **complete progressor (2≥20% increase)** | **5 (29%)** | **3 (21%)** | **1 (5%)** |

(continued)

| | **pMS-b** (n=17) | **pMS-nb** (n=14) | **HS** (n=20) |
|---|---|---|---|
| mean change in TW25F (95% CI) | 1.39 [-0.32 - 3.11] | 0.41 [0.23 - 0.6] | 0.29 [NA] |
| mean change in Po (95% CI) | -0.36 [-0.69 - -0.03] | -0.14 [-0.62-0.33] | -0.12 [NA] |

### References

[1] J. C. E. Kempen, C. A. M. Doorenbosch, D. L. Knol, V. de Groot, and H. Beckerman, "Newly Identified Gait Patterns in Patients With Multiple Sclerosis May Be Related to Push-off Quality," Phys.Ther., vol. 96, no. 11, pp. 1744-1752, Nov. 2016.

[2] L. Filli, Sutter T, Easthope CS, Killeen T, Meyer C, Reuter K, et al., "Profiling walking dysfunction in multiple sclerosis: characterisation, classification and progression over time," Scientific Reports, vol. 8, no. 1, p. 4984, Mar. 2018.

[3] K. J. Kelleher, W. Spence, S. Solomonidis, and D. Apatsidis, "The characterisation of gait patterns of people with multiple sclerosis," Disabil. Rehabil., vol. 32, no. 15, pp. 1242-1250, 2010.

[4] C. H. Polman et al., "Diagnostic criteria for multiple sclerosis: 2010 revisions to the McDonald criteria," Ann. Neurol., vol. 69, no. 2, pp. 292-302, Feb. 2011.

[5] N. J. Perkins and E. F. Schisterman, "The Inconsistency of 'Optimal' Cut-points Using Two ROC Based Criteria.," Am. J. Epidemiol., vol. 163, no. 7, pp. 670-675, Apr. 2006.

[6] W. J. Youden, "Index for rating diagnostic tests," Cancer, vol. 3, no. 1, pp. 32-35, Jan. 1950.

[7] J. M. Huisinga, K. K. Schmid, M. L. Filipi, and N. Stergiou, "Gait Mechanics Are Different Between Healthy Controls and Patients With Multiple Sclerosis," J. Appl. Biomech., vol. 29, no. 3, pp. 303-311, Jun. 2013.

[8] C. L. Brockett and G. J. Chapman, "Biomechanics of the ankle," Orthop. Trauma, vol. 30, no. 3, pp. 232-238, Jun. 2016.

[9] L. E. Cofre Lizama, F. Khan, P. V. Lee, and M. P. Galea, "The use of laboratory gait analysis for understanding gait deterioration in people with multiple sclerosis," Mult. Scler. Houndmills Basingstoke Engl., vol. 22, no. 14, pp. 1768-1776, 2016.

[10] Kurtzke JF. Disability rating scales in multiple sclerosis. Ann N Y Acad Sci 1984;436:347-60.

[11] Ruet A, Deloire M, Charré-Morin J, Hamel D, Brochet B. Cognitive impairment differs between primary progressive and relapsing-remitting MS. Neurology 2013;80:1501-8. doi:10.1212/WNL.0b013e31828cf82f.

[12] Fischer JS, Rudick RA, Cutter GR, Reingold SC. The Multiple Sclerosis Functional Composite measure (MSFC): an integrated approach to MS clinical outcome assessment. Mult SclerJ 1999;5:244-50. doi:10.1177/135245859900500409.

[13] Tinetti ME, Speechley M, Ginter SF. Risk factors for falls among elderly persons living in the community. N Engl J Med 1988;319:1701-7.

[14] Tourbah A, Lebrun-Frenay C, Edan G, Clanet M, Papeix C, Vukusic S, et al. MD1003 (high-dose biotin) for the treatment of progressive multiple sclerosis: A randomised, double-blind, placebo-controlled study. Mult Scler Houndmills Basingstoke Engl 2016;22:1719-31. doi:10.1177/1352458516667568.

[15] "Research Methods in Biomechanics," J. Sports Sci. Med., vol. 13, no. 1, p. i, Jan. 2014.

[16] C. P. Witana, S. Xiong, J. Zhao, and R. S. Goonetilleke, "Foot measurements from three-dimensional scans: A comparison and evaluation of different methods," Int. J. Ind. Ergon., vol. 36, no. 9, pp. 789-807, Sep. 2006.

**Claims**

1. Device for calculating, during one step or each successive step of the gait of a subject, the push-off Po of the subject, which is the power per kilogram released by the ankle push-off moment, which comprises:

   • at least one inertial measurement unit (1A, 1B) on one foot of the subject, the inertial measurement unit (1A, 1B) having: at least one accelerometer to measure the vertical and antero-posterior accelerations, and/or at least one gyroscope to measure the medio-lateral angular speed data $\dot{\alpha}$ during the gait,
   • calculation means (2A) connected to the Inertial measurement unit (1A, 1B), configured to calculate: for the foot, and for the step or each successive step of the gait:

   - the time of the heel-off and the time of the toe-off,
   - the push-off Po, by the Euler's equation stating that the sum of moments acting on the foot taken as a rigid body, being equal to the rate of change of the angular momentum of the foot,
   at the time of the toe-off when the sagittal angular momentum is at its maximum in absolute value,

   • displaying means (2B) connected to the calculation means (2A).

2. Device according to claim 1, wherein each inertial measurement unit (1A, 1B) has at least one gyroscope to measure the medio-lateral angular speed data $\dot{\alpha}$ during the gait, and the push-off Po is equal to :

$$\text{Po} = r_A \cdot g \cdot \cos\alpha \cdot \omega$$

   $\alpha$ being the integration of the medio-lateral angular speed data $\dot{\alpha}$ between the time of the heel-off and the time of the toe-off,
   $\omega$ being a value of the gyration medio-lateral angular speed data $\dot{\alpha}$,
   $r_A$ being the distance between the center of pressure to the tibio-talus articulation.

3. Device according to claim 2, wherein $\omega$ is the value of the gyration medio-lateral angular speed data $\dot{\alpha}$, at the time of the toe-off, at $\pm 10\%$.

4. Device according to claims 2- 3, wherein the device comprises at least one inertial measurement unit (1A, 1B) per foot of the subject,
   the calculation means (2A) calculate for each foot, with N natural number of successive steps greater than or equal to two:

   - $\alpha_i$, i varying from 1 to N successive steps of the gait;
   - $\omega_i$, i varying from 1 to N successive steps of the gait;
   - the mean $|\text{Po}| = r_A \cdot g \cdot \cos|\alpha| \cdot |\omega|$
   with $|\alpha|$ the mean of $\alpha i$, and $|w|$ the mean of $wi$;
   and the calculation means (2A) calculate $P_0$ which is function of: the push-off of the right foot $|P_{0\ right}|$ and the push-off of the left foot $|P_{0\ left}|$.

5. Device according to claim 4, wherein $P_0$ is the minimum of $|P_{0\ right}|$ and $|P_{0\ left}|$.

6. Device according to claims 4-5, wherein N is comprised between to 5 to 20, for a 10-meter walking exercise.

7. Device according to claims 1-6, wherein the device is intended to be embedded on the subject, the calculation means (2A) and the displaying means (2B) being an embedded support (3) which communicates with the inertial measurement unit (1A, 1 B) using WIFI or Bluetooth®.

8. Device according to claim 7, wherein the embedded support (3) is a computer, a tablet, a smartphone with an Application for gait quantification.

9. System which comprises:

   - the device for calculating the push-off $P_0$ according to claims 1-8,
   - a rehabilitation device of the subject linked to the device for calculating the push-off $P_0$ according to claims 1-8.

10. System according to claim 9, wherein the rehabilitation device of the subject is:

   • Functional Electrical Stimulation technologies ;
   • Mechanical and electronical ankle-foot orthosis ;
   • Robotic prosthesis and exoskeleton ;
   • Biofeedback electrical device ;
   • Connected insoles, shoes, socks or elastic supportive hoses;
   • Continuous Passive Motion medical devices

11. Method to calculate, during one step or each successive step of the gait of a patient, the push-off $P_0$ of the subject, which is the power per kilogram released by the ankle push-off moment,
   from the vertical and antero-posterior accelerations data and/or the medio-lateral angular speed data $\dot{\alpha}$ of the patient for one step or several successive steps of a gait, and by using the device for calculating the push-off $P_0$ according to claims 1-8,
   comprising the following steps:

   - (i) calculating the time of the heel-off and the time of the toe-off of the step or each successive step;
   - (ii) calculating for the foot, the push-off $P_0$,
   by the Euler's equation stating that the sum of moments acting on the foot taken as a rigid body, being equal to the rate of change of the angular momentum of the foot, at the time of the toe-off when the sagittal angular momentum is at its maximum in absolute value.

12. Method according to claim 11, wherein each Inertial measurement unit (1A, 1B) having at least one gyroscope to measure the medio-lateral angular speed data $\dot{\alpha}$ during the gait,
   and the push-off $P_0$ is equal to :

$$\text{Po} = r_A \cdot g \cdot \cos \alpha \cdot \omega$$

   $\alpha$ being the integration of the medio-lateral angular speed data $\dot{\alpha}$ between the time of the heel-off and the time of the toe-off,
   $\omega$ being a value of the gyration medio-lateral angular speed data $\dot{\alpha}$,
   $r_A$ being the distance between the center of pressure to the tibio-talus articulation.

13. Method according to claim 12, wherein $\omega$ is the value of the gyration medio-lateral angular speed data $\dot{\alpha}$ at the time of the toe-off at $\pm10\%$.

14. Method according to claims 11-13, wherein:
   in the step (ii) calculating for each foot, with N natural number greater than or equal to two:

   - $\alpha_i$, i varying from 1 to N successive steps of the gait;
   - $\omega_i$, i varying from 1 to N successive steps of the gait;
   - the mean $|\text{Po}| = r_A \cdot g \cdot \cos|\alpha| \cdot |\omega|$
   with $|\alpha|$ the mean of $\alpha i$, and $|w|$ the mean of $wi$;
   and calculating $P_0$ which is function of: the push-off of the right foot $|P_{0\ right}|$ and the push-off of the left foot $|P_{0\ left}|$.

15. Method according to claims 9-13, used to analyse/predict fall risk in patient, and comprises an additional step:

   - (iii) with the value of the push-off $P_0$ compared to a threshold, displaying the fall risk of the patient, the fall risk being high compared to the fall risk of a reference healthy subject if the value of the push-off $P_0$ is inferior to the threshold.

**16.** Method according to claim 14-15, wherein in the step (iii), the threshold is fixed with several patients so as to:

- maximize (sensitivity + specificity -1) ;
- the sensitivity is superior to 90%;

**17.** Method according to claim 16, wherein in the step (iv), the threshold is 6.9 W/kg for patients with multiple sclerosis.

**18.** Method according to claim 15, wherein the threshold is a previous value of the push-off of the patient calculated 6 months before, and a change of ≥20% is considered significant for all populations of patients.

**19.** Method according to claims 11-14, used to evaluate a treatment, by calculating, during each successive step of the gait of patients who follow the treatment, comprising the additional step:

- (iii) display the time evolution of the push-off the patients,

• an increase in the push-off $P_0$ decreasing the fall risk of the patient,
the treatment device being validated if the increase of the push-off $P_0$ is superior or equal to 20%,
• a decrease in the push-off $P_0$ increasing the fall risk of the patient,
the treatment device being unvalidated if the decrease of the push-off $P_0$ is inferior or equal to 20%.

**20.** Method according to claim 19, wherein the treatment is realized with a medication and/or a rehabilitation device and/or a rehabilitation program

FIG. 1A

FIG. 1B

FIG. 2A

EP 3 731 238 A1

$$\overline{\alpha_{right}} = \frac{\sum_{i=1}^{13} \alpha_i}{13}$$

$$\overline{\omega_{right}} = \frac{\sum_{i=1}^{13} \omega_i}{13}$$

$$\overline{\alpha_{left}} = \frac{\sum_{i=1}^{14} \alpha_i}{14}$$

$$\overline{\omega_{left}} = \frac{\sum_{i=1}^{14} \omega_i}{14}$$

$$P0_{right} = r_\Lambda.g.\cos\overline{\alpha_{right}} \times \overline{\omega_{right}}$$

$$P0_{left} = r_\Lambda.g.\cos\overline{\alpha_{left}} \times \overline{\omega_{left}}$$

$$P0 = \min(P0_{right}, P0_{left})$$

FIG. 2B

FIG. 3A

FIG. 3B

FIG. 3C

| M0 | | M6 | | M12 |

16 pMS
(progressive pMS
with no change
of treatment)

20 HS
(healthy subjects
from hospital and
research unit staff)

8 F-pMS
8 NF-pMS

0 F-HS
20 NF-HS

6 F-pMS
9 NF-pMS

0 F-HS
20 NF-HS

# FIG. 4

ICC Force Plates versus XSens - Po of individual step

● left foot: ICC(1,1) = 0.79 , ICC(3,1) = 0.79
● right foot: ICC(1,1) = 0.76 , ICC(3,1) = 0.76

ICC Force Plates versus XSens - Po

○ minimum of right and l+ foot: ICC(1,1) = 0.74 , ICC(3,1) = 0.74
● left foot: ICC(1,1) = 0.76 , ICC(3,1) = 0.76
● right foot: ICC(1,1) = 0.49 , ICC(3,1) = 0.83

FIG. 5A

FIG. 5B

FIG. 6A

FIG. 6B

FIG. 7A

**4-fold validation ROC curve**

T25FW$_{th}$ = 11.7 s
Se: 90.0
Sp: 47.3

T25FW$_{th}$ = 11.7 s
Se: 86.2
Sp: 53.5

P$_{o th}$ = 6.9 W/kg
**Se: 90.0**
Sp: 60.9

P$_{o th}$ = 6.8 W/kg
**Se: 78.0**
Sp: 81.8

— AUC= 0.85 [0.73 - 0.95]
---- AUC= 0.75 [0.6 - 0.89]

Se

1-Sp

**ROC curve on the test set**

T25FW$_{th}$ = 11.7 s
Se: 92.1
Sp: 82.8

T25FW$_{th}$ = 11.7 s
Se: 87.0
Sp: 57.8

P$_{o th}$ = 6.9 W/kg
Se: 90.9
Sp: 72.8

P$_{o th}$ = 6.8 W/kg
Se: 78.6
Sp: 89.1

— AUC= 0.94 [0.85 - 1]
---- AUC= 0.95 [0.72 - 0.97]
p-value (Y) <.0001
p-value (Yc) <.0001
p-value (AUC) <.0001

Se

1-Sp

FIG. 7B

FIG. 8

FIG. 9

Difference in fall number
between M6-M12 and M0-M6

▽ lower
○ equal
△ higher

R: complete responder

FIG. 10B

Difference in fall number
between M6-M12 and M0-M6

▽ lower
○ equal
△ higher

R: complete responder

FIG. 10A

FIG. 11

EP 3 731 238 A1

**Europäisches Patentamt**
**European Patent Office**
**Office européen des brevets**

# EUROPEAN SEARCH REPORT

Application Number

EP 19 17 0581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/060512 A1 (GREENE BARRY R [IE]) 7 March 2013 (2013-03-07) * paragraphs [0001], [0030] - [0039], [0055] - [0057]; figure 10 * | 1-20 | INV. G16H50/30 A61B5/11 G16H50/70 G16H20/30 G16H20/10 |
| X | US 2018/279915 A1 (HUANG MING-CHUN [US] ET AL) 4 October 2018 (2018-10-04) * paragraphs [0014] - [0022] * | 1-20 | |
| X | US 2017/197115 A1 (COOK JAMES BINGHAM [GB] ET AL) 13 July 2017 (2017-07-13) * paragraphs [0013], [0022] * | 1-20 | |

TECHNICAL FIELDS SEARCHED (IPC)

G16H
A61B

The present search report has been drawn up for all claims

| Place of search | Date of completion of the search | Examiner |
|---|---|---|
| Munich | 16 October 2019 | Reinbold, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 0581

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2019

| Patent document cited in search report | | Publication date | Patent family member(s) | | Publication date |
|---|---|---|---|---|---|
| US 2013060512 | A1 | 07-03-2013 | NONE | | |
| US 2018279915 | A1 | 04-10-2018 | CN | 109069066 A | 21-12-2018 |
| | | | EP | 3355783 A1 | 08-08-2018 |
| | | | US | 2018279915 A1 | 04-10-2018 |
| | | | WO | 2017058913 A1 | 06-04-2017 |
| US 2017197115 | A1 | 13-07-2017 | EP | 3403206 A1 | 21-11-2018 |
| | | | JP | 2019508191 A | 28-03-2019 |
| | | | US | 2017197115 A1 | 13-07-2017 |
| | | | WO | 2017118908 A1 | 13-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Percy Hospital,* December 2017
- Protection des Personnes Nord Ouest III. *Helsinki principles and approved by Ethic Comity,* 2017, A01538-45
- **J. C. E. KEMPEN ; C. A. M. DOORENBOSCH ; D. L. KNOL ; V. DE GROOT ; H. BECKERMAN.** Newly Identified Gait Patterns in Patients With Multiple Sclerosis May Be Related to Push-off Quality. *Phys. Ther.,* November 2016, vol. 96 (11), 1744-1752
- **L. FILLI ; SUTTER T ; EASTHOPE CS ; KILLEEN T ; MEYER C ; REUTER K et al.** Profiling walking dysfunction in multiple sclerosis: characterisation, classification and progression over time. *Scientific Reports,* March 2018, vol. 8 (1), 4984
- **K. J. KELLEHER ; W. SPENCE ; S. SOLOMONIDIS ; D. APATSIDIS.** The characterisation of gait patterns of people with multiple sclerosis. *Disabil. Rehabil.,* 2010, vol. 32 (15), 1242-1250
- **C. H. POLMAN et al.** Diagnostic criteria for multiple sclerosis: 2010 revisions to the McDonald criteria. *Ann. Neurol.,* February 2011, vol. 69 (2), 292-302
- **N. J. PERKINS ; E. F. SCHISTERMAN.** The Inconsistency of 'Optimal' Cut-points Using Two ROC Based Criteria. *Am. J. Epidemiol.,* April 2006, vol. 163 (7), 670-675
- **W. J. YOUDEN.** Index for rating diagnostic tests. *Cancer,* January 1950, vol. 3 (1), 32-35
- **J. M. HUISINGA ; K. K. SCHMID ; M. L. FILIPI ; N. STERGIOU.** Gait Mechanics Are Different Between Healthy Controls and Patients With Multiple Sclerosis. *J. Appl. Biomech.,* June 2013, vol. 29 (3), 303-311
- **C. L. BROCKETT ; G. J. CHAPMAN.** Biomechanics of the ankle. *Orthop. Trauma,* June 2016, vol. 30 (3), 232-238
- **L. E. COFRE LIZAMA ; F. KHAN ; P. V. LEE ; M. P. GALEA.** The use of laboratory gait analysis for understanding gait deterioration in people with multiple sclerosis. *Mult. Scler. Houndmills Basingstoke Engl.,* 2016, vol. 22 (14), 1768-1776
- **KURTZKE JF.** Disability rating scales in multiple sclerosis. *Ann N Y Acad Sci,* 1984, vol. 436, 347-60
- **RUET A ; DELOIRE M ; CHARRÉ-MORIN J ; HAMEL D ; BROCHET B.** Cognitive impairment differs between primary progressive and relapsing-remitting MS. *Neurology,* 2013, vol. 80, 1501-8
- **FISCHER JS ; RUDICK RA ; CUTTER GR ; REINGOLD SC.** The Multiple Sclerosis Functional Composite measure (MSFC): an integrated approach to MS clinical outcome assessment. *Mult Scler J,* 1999, vol. 5, 244-50
- **TINETTI ME ; SPEECHLEY M ; GINTER SF.** Risk factors for falls among elderly persons living in the community. *N Engl J Med,* 1988, vol. 319, 1701-7
- **TOURBAH A ; LEBRUN-FRENAY C ; EDAN G ; CLANET M ; PAPEIX C ; VUKUSIC S et al.** MD1003 (high-dose biotin) for the treatment of progressive multiple sclerosis: A randomised, double-blind, placebo-controlled study. *Mult Scler Houndmills Basingstoke Engl,* 2016, vol. 22, 1719-31
- Research Methods in Biomechanics. *J. Sports Sci. Med.,* January 2014, vol. 13 (1), i
- **C. P. WITANA ; S. XIONG ; J. ZHAO ; R. S. GOONETILLEKE.** Foot measurements from three-dimensional scans: A comparison and evaluation of different methods. *Int. J. Ind. Ergon.,* September 2006, vol. 36 (9), 789-807